# EUROPEAN PATENT SPECIFICATION

(11) **EP 0 530 937 B1**
(45) Date of publication and mention of the grant of the patent: **12.08.1998**
(21) Application number: 92250245.5
(22) Date of filing: 04.09.1992
(51) Int. Cl.: C12N 15/12, C12P 21/02, C07K 14/00, A61K 38/17, C12N 5/10

(54) **Collagen-induced platelet aggregation inhibitor**
Hemmstoff der Kollagen-induzierten Plättchenaggregation
Inhibiteur de l'aggrégation plaquettaire induit par le collagène

(30) Priority: 05.09.1991 US 756211; 31.12.1991 US 814884; 17.07.1992 US 924383
(43) Date of publication of application: 10.03.1993
(73) Proprietor: SCHERING AKTIENGESELLSCHAFT, 13353 Berlin (DE)
(72) Inventor: Noeske-Jungblut, Christiane, W-1000 Berlin 39 (DE); Haendler, Bernhard, W-1000 Berlin 12 (DE); Krätzschmar, Jörn, W-1000 Berlin 30 (DE); Schleuning, Wolf-Dieter, W-1000 Berlin 20 (DE); Alagon, Alejandro, Cuernavaca, Morelos 62271 (MX); Possani, Lourival, Cuernavaca, Morelos 62271 (MX); Cuevas-Aguirre, Delia, Cuernavaca, Morelos 62271 (MX)

(56) References cited:
- EXPERIENTIA vol. 37, 1981, BASEL CH pages 384 - 386 J.M.C. RIBEIRO ET AL. 'Platelet antiaggregating activity in the salivary secretion of the blood sucking bug Rhodnius porlixus'
- BIOLOGICAL ABSTRACTS vol. 76 , 1983, Philadelphia, PA, US; abstract no. 42969, G.J. GASIC ET AL. 'Inhibition of lung tumor colonization by leech salivary gland extracts from Haementeria ghilianii'

## Description

### STATE OF ART

Collagen is the most potent inducer known of human platelet aggregation. For instance, upon injury of the vessel wall and exposure to collagen, blood platelets rapidly adhere and become activated. (BAUMGARTNER, H.R. (1977) Thromb. Haemostas. **37**, 1-16; HAWIGER, J. (1987) Human Pathol. **18**, 111-122.)

Collagen-induced platelet aggregation of human platelets thus represents a risk factor for patients undergoing blood vessel-affecting procedures, e. g., angioplasty or sepsis, for those suffering myocardial infarction, for those recovering from treatment for myocardial infarction, *inter alia*.

Inhibitors of the collagen-induced platelet aggregation include synthetic oligopeptides corresponding to a collagen sequence, a snake venom protein and calin, a protein from the medicinal leech. The synthetic oligopeptides inhibit the collagen-induced platelet aggregation by binding to the platelets. A disadvantage is that they have an effect on platelet aggregation only in a relatively high dosage (about 70 µg/ml of the peptide yields 65% inhibition). See BEVERS et al. (1985) "Collagen Derived Octapeptide Inhibits Platelet Procoagulant Activity Induced by the Combined Action of Collagen and Thrombin", Thrombosis Research, **37**; 365-370, KARNIGUIAN et al.(1983) "Effect of a Collagen Derived Octapeptide on Different Steps of the Platelet/Collagen Interaction", Thrombosis Research **32**:593-604, and CAEN et al. (1981) "Oligopeptides with specific inhibiting properties of collagen-induced aggregation, process for preparing the same and pharmaceutical compositions containing them", EPA 0 040 149. The inhibitory mechanism of the snake venom protein is still unknown. See SMITH et al., "Identification of 50 kDalton snake venom proteins which specifically inhibit platelet adhesion to collagen." Thrombosis and Haemostasis (1991, **65**, 678 (abstracts of the XIIIth Congress of the International Society on Thrombosis and Haemostasis, June 30 - July 6, 1991 in Amsterdam). Calin reacts with collagen; it does not react with platelets. Thus, it is not specific for the platelet-collagen interaction, but it reacts also with collagen in the absence of platelets. See MUNRO et al.(1991) "Calin - a platelet adhesion inhibitor from the salvia of the medicinal leech", Blood Coagulation and Fibrinolysis **2**, 179-184.

The publication of the European patent application EP 0 480 651 (Merck & Co. Inc. published 15 April, 1992) describes a protein having a molecular weight of about 16 kDalton and a capacity to inhibit collagen-induced aggregation of human platelets which protein is derived from the salivary gland of the leech *Haemaenteria officinalis*.

Other inhibitors of such aggregation are needed having varied or improved properties.

The invention provides a natural isolated, synthetically manufactured or recombinant protein which inhibits collagen-induced aggregation of mammalian platelets and which is isolated or isolatable from saliva of mammalian-blood sucking insects.

Primate platelets are more preferred, most preferred are human platelets. The platelets from other species are also sensible, e.g., horses, sheep, cattle, pigs, dogs and cats.

Synthetically manufactured proteins can be prepared according to J.M. STEWARD and J.D. YOUNG (1984) Solid Phase Peptide Synthesis, Pierce Chem. Company, Fockford III and according to Methoden der Organischen Chemie (HOUBEN/WEYL), Vol 15/No. 1 and 2, E. WÜNSCH (ed.), Thieme Verlag Stuttgart, 1974.

Preferred is a protein of the invention which is isolated or isolatable from saliva of the subfamily (lat. Faminia) *Triatomae and* more preferred of *Triatoma pallidipennis*.

The invention comprises a natural isolated, synthetically manufactured or recombinant protein which inhibits collagen-induced aggregation of mammalian platelets and
which protein has a N-terminal amino acid sequence

Glu · Glu · Cys · Glu · Leu · Met · Pro · Pro · Gly · Asp · Asn · Phe · Asp · Leu ·
1 5 10 14

Glu · Lys · Tyr · Phe · Ser · Ile.
15 20

A further embodyment of the invention is a protein which inhibits collagen-induced aggregation of mammalian platelets
which protein has the following amino acid sequence:
a) the sequence in
   aa) Seq. Id. No. 1,
   bb) Seq. Id. No. 2 or
   cc) Seq. Id. No. 3
   or
b) allelic modifications or muteins of the sequences in anyone of the Seq. Ids No. 1 to 3 which allelic modifications or muteins do not substantially affect the activity of the protein,
   or
c) a protein according to anyone of the Seq. Ids No. 1 to 3 or their modifications or muteins mentioned under b) comprising posttranslational modifications, which do not substantially affect the activity of the mature protein.

More preferred is a protein as mentioned before which is a recombinant protein.

The invention comprises a protein which is free of glycosylation.

A futher embodyment of the invention is a cDNA or DNA
a) coding for a protein which has the following amino acid sequence:
   a) the sequence in
      aa) Seq. Id. No. 1,
      bb) Seq. Id. No. 2 or
      cc) Seq. Id. No. 3
   or
b) coding for a protein which has a sequence of amino acids according to anyone of the Seq. No. 1 to 3 with at least one allelic modification or mutein which does not substantially affect the activity of the mature protein encoded by the corresponding cDNA or DNA sequence.

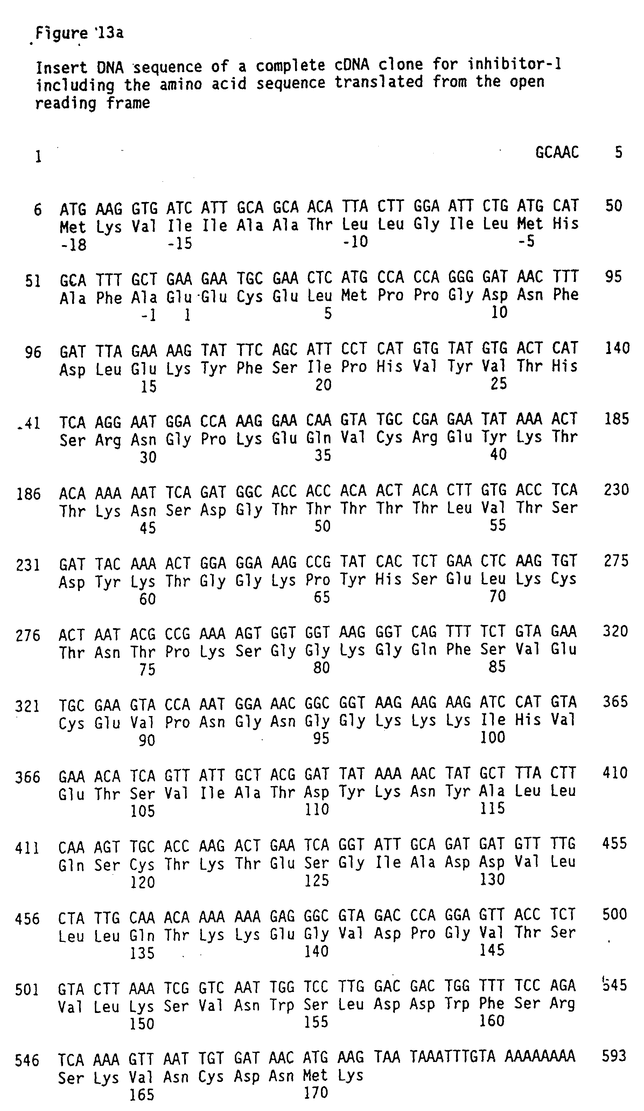

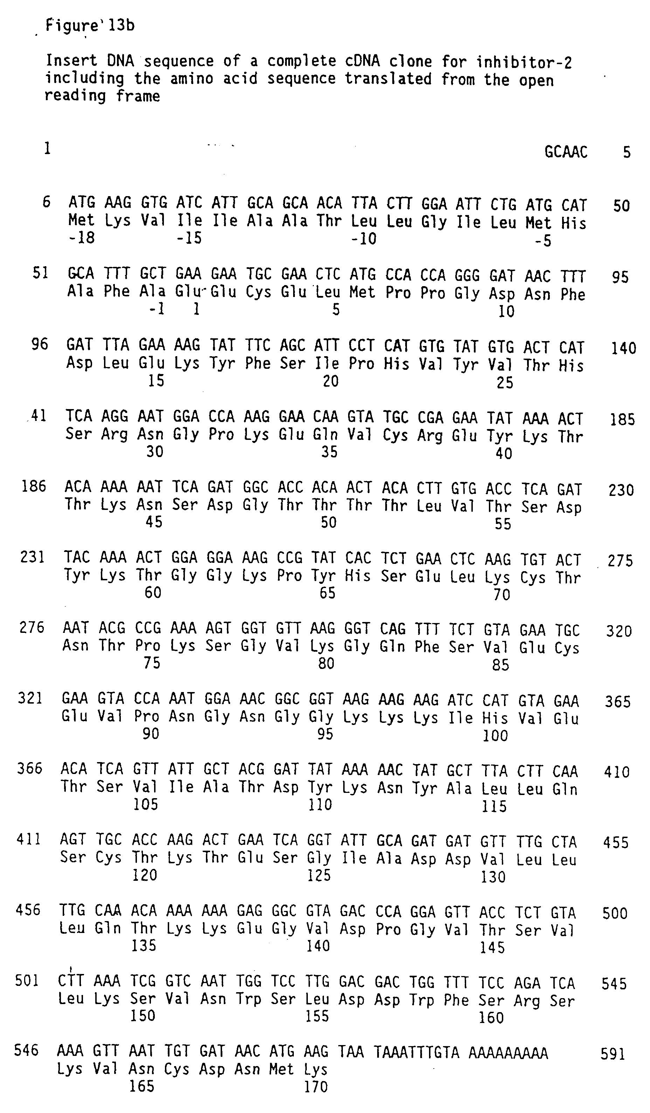

The protein of the invention comprises a mature protein and a preprotein which has as signal sequence preceeding the N-terminal part of the mature protein. The signal sequences can be seen in Figures 13a and 13b and they can be recognized by their negative enumeration. It starts with Met · Lys · Val · Ile · Ile · and ends with · His · Ala · Phe · Ala. The signal sequence is responsible for the penetration of the membran after protein biosynthesis. The protein, which is secreted, is the mature protein, starting with Glu · Glu · Cys · Glu · Leu · .... The signal sequence is cleaved prior to secretion..

The invention preferrably comprises a cDNA or DNA with the following nucleotide sequence:
a) the nucleotide sequence in
   aa) Seq. Id. No. 4,
   bb) Seq. Id. No. 5 or
   cc) Seq. Id. No. 6
   or
b) a sequence of nucleotides according to any one of the Seq. No. 4 to 6 with at least one allelic modification or mutein which does not substantially affect the activity of the mature protein which is encoded by the corresponding nucleotide sequence.

A further part of the invention is a vector comprising a cDNA or DNA as mentioned before, further comprising a suitable signal peptide, a suitable promoterand, if need be, a suitable enhancer. Vectors are described in detail in the literature of the Examples and also in the European publications EP 0 480 651; 0 462 632 and 0 173 177.

A further embodyment of the invention is an eukaryotic or prokaryotic host cell transformed with a vector as mentioned above.

The most preferred host cell is a baby hamster kidney cell.

The invention additionally comprises a method of producing a protein according to the invention which method comprises
culturing a host cell transformed by a vector comprising the gene coding for the protein
and
isolating and purifying the protein. The concrete embodyments are described in the Examples of the invention, the general method can be deduced from the state of the art mentioned in the specification, especially in the Examples of the invention.

The industrial application of the proteins of the invention is the use of the proteins as a pharmaceutical composition comprising a protein according to the invention in association with pharmaceutically acceptable dilutent or carrier. Details are mentioned in the part UTILIY of the specification.

Allelic modifications as mentioned before comprise alteration in the sequence of the nucleotides or amino acids, alteration of the genotype or phenotype. At least one nucleotide or one amino acid can be substitued, deleted or inserted.

Most deletions, insertions and substitutions in particular, are not expected to produce radical changes in the characteristics of the protein of the invention. As it is difficult to predict the exact effect of the substitution, deletion, or insertion in advance, the comparison ot the functions of the mutated protein with the characteristic functions of the protein of the invention clarify whether the altered protein has a comparable activity.

The genetic code is degenerated; that is, most amino acids are coded for by more than one codon of three nucleotides. Accordingly, allelic variation in the nucleotide sequence may or may not alter the amino acid sequence. Therefore, allelic variations are primarily on the DNA level and may also exist secondarily on the level of the amino acid sequence.

The DNA sequence coding for the protein of the invention can be modified by conventional techniques to produce variations in the final protein of the invention which still has substantially the same acitivity as the protein of the invention. The acitivity is measured according to Example 1. Thus, one or more amino acids, for example 1,2,3,4,5,6,7,8,9,10...up to 15 amino acids, can be added, substituted or removed without substantially affecting the activity of the protein of the invention. Substitutions can generally be made in accordance with the following Table 1 when it is desired to modulate finely the amino acid sequence of the protein of the invention.

Substantial changes in function or immunological identity are made by selecting substitutions that are less conservative than those in Table 1, i.e. selecting residues that differ more significantly in their effect on maintaining (a) the structure of the polypeptide backbone in the area of the substitution, for example, as a sheet or helical conformation, (b) the charge or hydrophobicity of the molecule, or (c) the bulk of the side chains.

**TABLE 1**

| NORMAL SUBSTITUTIONS OF AMINO ACIDS IN A PROTEIN | |
|---|---|
| ORIGINAL RESIDUES | EXEMPLARY SUBSTITUTIONS |
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Muteins are defined by homology between two compared proteins. The expression homology comprises similarities of the amino acids and gaps in the sequences of both compared sequences. Similarity of amino acids is defined for example in Table 1.

Preferably the protein has a sequence of amino acids having a homology of at least 60%, more preferred at least 80 %, much more preferred at least 90% and most preferred at least 95% of the sequence shown in one of the Sequence Identifiers 1 to 3. .

As mentioned before the invention comprises variation of the DNA. These sequences hybridise under stringent condition to the DNA sequence defined in one of the Sequency Identifiers 4 to 6. Preferably the cDNA or DNA has a sequence of nucleotides having a homology of at least 60%, more preferred at least 80 %, much more preferred at least 90% and most preferred at least 95% of the sequence shown in one of the Sequence Identifier 4 to 6. The homology can be measured by hybridisation described in R. KNIPPERS, Molekulare Genetik, 1982, third edition, Georg Thieme Verlag Stuttgart, New York..

By "post-translational varations" as mentioned above is meant varations during or after translation, such as glycosylation, formation of disulfide bridges and chemical modifications of amino acids.

Glycosylation is one of the major biosynthetic functions of the endoplasmic recticulum and/or Golgi apparatus. The sequence and branching of the oligosaccharides formed in the reticulum can be altered in the Golgi apparatus, the lysosomes or plasma membrane. The oligosaccharides can be N-linked oligosaccharides (asparagine linked) or O-linked oligosaccharides (serine, threonine or hydroxylysine linked). The glycosylation is dependent on the producing cell type and the species from which the cell type derives. The amount and form of glycosylation can be influenced by compounds as described in European Patent Application EP 0 222 313. Changes in glycosylation may affect the function of the protein.

Normally the mature protein of the invention is glycosylated.

Proteins often form covalent intrachain bonds. These disulfide bonds are formed between cysteine-SH amino acids in the folded protein or in the protein which folds during translation. The bonds stabilize the three-dimensional structure of the protein. Such disulfide bonds are rarely formed in protein molecules that are still in the cell cytosol because the high intracellular concentration of the -SH reducing agent glutathione breaks most of such bonds.

Once the proteins are outside the cytoplasm, are secreted or are on the cell surface, they often form additional covalent intrachain bonds.

Furthermore, the amino acids may be altered as described in PCT Application WO 91/10684. Other alterations of the side chains of the amino acids are possible.

A preferred homology is at least 90%, more preferred at least 95%, much more preferred at least 98% and most preferred the alteration of one or two amino acids.

The protein of the invention has at least a purity of 40%, preferably at least 60%, more preferably at least 80% and most preferably at least 90%. The purity is defined by the amount of the protein of the invention in relation to the total amout of protein. Using the purification in two steps, first by Sepharose gel filtration (see Example 2) and second by HPEC (see Example 15) no other protein beside the proteins of the invention are detectable by methods described in the Example 15.

Further, the invention comprises binding molecules, single chain proteins, antibodies or fragments thereof, recognizing specifically domains on the mature protein of the invention.

Using the purified protein of the invention (see for example the sequences of Seq Ids No. 1 to 3) the monoclonal antibodies are produced according to the well-known Koehler and Milstein method which, in particular, comprises conventionally immunizing mice with the purified protein of the invention as immunogen.

The best mode of the invention is the protein mentioned in Sequence Identifier No. 1, expressed in transfected baby hamster kidney cells.

The invention additionally comprises a method of purification of the protein of the invention comprising (a) a step of gel filtration using "Superose 12" (See Example 2) and (b) a step of High Performance Electrophoresis Chromatography System (See Example 15).

### UTILITY OF THE COMPOUNDS

The proteins of the invention exhibit pharmacological activity and may, therefore, be useful as pharmaceuticals. They can be used in a pharmaceutical composition comprising a protein of the invention in association with pharmaceutically acceptable diluent or carrier. Additionally the invention comprises a pharmaceutical composition comprising a pharmaceutically acitve protein according to the invention and a pharmaceutically acceptable salt or a pharmaceutically acceptable carrier.

In particular, the protein of the invention shows inhibition of collagen-induced platelet aggregation and inhibition of adhesion of tumor cells, preferred of metastatic tumor cells, to collagen.

### Medicament against platelet aggregation

The proteins of the invention show inhibition of platelet aggregation. The test system is described in Example 1. The proteins of the invention show a significant inhibition of platelet aggregation in a concentration of 0.5 to 50 µg protein saliva in 0.5 ml or 0.5 to 250 µg protein in 0.7 ml of purified protein (only purified according Example 2).

The test of the most preferred protein, the protein of Sequence Identifier 1, shows a value of the IC₅₀ of 50 nmol/l of highly purified protein according to the Examples 2 and 15. The proteins of the invention show the inhibition of platelet aggregation at concentrations of from 5 nmol/l to about 1,000 nmol/l.

The results from the *in vitro* test systems indicate that the proteins of the invention can be used as a medicament or can be used for medical treatment. The test results can be transferred from the *in vitro* system to the *in vivo* system, because it is an established system in this field. R.J. SHEBUSKI et al. (1990) Thrombosis and Haemostasis, **64**: 576 - 581

The proteins of the invention are administered by intraperitoneal injections which are given daily or at 2 to 3 times a week. When animals receive daily injections to achieve a blood concentration of 100 nmol/l, they have a reduced platelet aggregation.
No serious side effect are monitored under these conditions.

The proteins of the invention show this inhibition of platelet aggregation in mice at daily dosages to achieve a blood concentration of from about 10 nmol/l to 1,000 nmol/l.

The proteins of the invention are, therefore, useful for the treatment of atherosclerotic or thrombotic disease lesions or for preventing reocclusion after treatment of myocardial infarction. In other words: The proteins of the invention can be used as an antiatherosclerotic and antithrombotic agent in mammals, including humans, e.g., to treat atherosclerotic/thrombotic lesions, for example due to rupture of atherosclerotic plaques or those due to pertubation or removal of endothelium, e.g., in sepsis or transplants or to treat unstable angina. It can also be used to prevent reocclusion after treatment of myocardial infarction by fibrinolysis or by angioplasty (PTCA). If fibrinolytic therapy (with streptokinase, t-PA or other plasminogen activators) is applied to treat myocardial infarction the proteins of the invention can be used as an adjuvant agent to prevent reocclusion of the blood vessel. Treatment of myocardial infarction with a balloon catheter (PTCA) also injures the vessel wall and this may lead to formation of a new thrombus. This can be prevented by administering the proteins of the invention during and after the procedure. The proteins cannot be used only in coronary angioplasty but are also employable in other angioplasty applications.

The invention provides
a) the use of a protein of the invention for manufacture of a medicament for treatment of atherosclerotic or thrombotic disease or for preventing reocclusion after treatment of myocardial infarction; (The proteins are useful for prophylactically working medicaments.)
b) a method of treatment of atherosclerotic or thrombotic disease or for preventing reocclusion after treatment of myocardial infarction, which comprises administering of a disease-suppressing effective amount of the protein of the invention to a patient in need of such treatment;
c) a pharmaceutical composition for treatment of atherosclerotic or thrombotic disease or for preventing reocclusion after treatment of myocardial infarction which comprises a protein of the invention and a pharmaceutically acceptable carrier or diluent.

For these indications the appropriate dosage will, of course, vary depending upon, for example, the compound of the invention employed, the host, the mode of adminstration and the nature and severity of the condition being treated. However, in general, satisfactory results in animals are indicated to be obtained at daily dosages to achieve a blood concentration of from 10 to 1,000 nmol/l, preferred at daily dosages of from 30 to 300 nmol/l.

The proteins of the invention may be adminstered by any conventional route, in particular enterally, orally, e.g. in the form of tablets or capsules or parenterally, e.g. in the form of injectable solutions or suspensions.

The protein of the Sequence Identifier 1 is the preferred compound.

The present invention provides pharmaceutical compositions comprising compounds of the invention in association with at least one pharmaceutical carrier or diluent. Such compositions may be manufactured in conventional manner. See Remington's Pharmaceutical Science, 15^{th} ed. Mack Publishing Company, Easton Pennsylvania (1980).

### Medicament against metastatic tumor cells

The proteins of the invention show adhesion-inhibition of metastatic tumor cells to collagen. The test system is described in Example 14. The proteins of the invention show a significant adhesion-inhibition of metastatic tumor cells to collagen in a concentration of 1 to 100 µg protein saliva in 0.5 ml or 1 to 500 µg protein in 0.5 ml of purified protein (only purified according Example 2).

The test of the most preferred protein, the protein of Sequence Identifier 1, shows a value of the IC₅₀ of 100 nmol/l of the highly purified protein according to the Examples 2 and 15. The proteins of the invention show the adhesion-inhibition of metastatic tumor cells to collagen at concentration of from 10 to 2,000 nmol/l.

The results from the *in vitro* test systems indicate that the proteins of the invention can be used as a medicament or can be used for medical treatment. The test results can be transferred from the *in vitro* system to the *in vivo* system, because it is an established system in this field. Chan et al. (1990), Science, **2**: 1600 - 1602.

The proteins of the invention can be administered during and after surgical operations of the primary tumor to prevent formation of metastasis by detached tumor cells which may enter the blood stream during operation. These antimestastatic effects can be investigated in an "experimental" and "spontaneous" animal model as described by Chan et al. (1990), Science, **2**: 1600 - 1602. The proteins of the invention are administered by intraperitoneal injections which are given daily or at 2 to 3 times a week. When animals receive daily injections to achieve a blood concentration of 200 nmol/l, they have a reduced adhesion of metastatic tumor cells measured by counting the value of centers of setteled metastatic cells.
No serious side effect are monitored under these conditions.

The proteins of the invention show this adhesion-inhibition of metastatic tumor cells to collagen in mice at daily dosages to achieve a blood concentration of from 20 to 2,000 nmol/l, preferred concentrations of from 60 to 600 nmol/l.

The proteins of the invention are, therefore, useful for the treatment of cancer, preferred of cancer with metastatic tumor cells, most preferred of cancer with highly metastatic tumor cells.

The invention provides
a) the use of a protein of the invention for manufacture of a medicament for treatment of cancer with metastatic tumor cells. (The proteins are useful for prophylactically working medicaments administered before e.g., surgical removement of tumors.)
b) a method of treatment of cancer with metastatic tumor cells, which comprises administering of a disease suppressing effective amount of the protein of the invention to a patient in need of such treatment;
c) a pharmaceutical composition for treatment of cancer with metastatic tumor cells which comprises a protein of the invention and a pharmaceutically acceptable carrier or diluent.

For these indications the appropriate dosage will, of course, vary depending upon, for example, the compound of the invention employed, the host, the mode of adminstration and the nature and severity of the condition being treated. However, in general, satisfactory results in animals are indicated to be obtained at daily dosages to achieve a blood concentration of from 20 to 2,000 nmol/l, preferred at daily dosages of 60 to 600 nmol/l.

The proteins of the invention may be adminstered by any conventional route, in particular enterally, orally, e.g. in the form of tablets or capsules or parenterally, e.g. in the form of injectable solutions or suspensions.

The protein of the Sequence Identifier 1 is the preferred compound.

The present invention provides pharmaceutical compositions comprising compounds of the invention in association with at least one pharmaceutical carrier or diluent. Such compositions may be manufactured in conventional manner. See Remington's Pharmaceutical Science, 15^{th} ed. Mack Publishing Company, Easton Pennsylvania (1980).

### SUMMARY OF THE INVENTION

This invention provides an inhibitor of the collagen-induced aggregation of human platelets. The new specific inhibitor is naturally occuring, is a protein (i.e., not an oligopeptide) and also inhibits the tumor cell-collagen interaction. Such an inhibitor is present in the saliva of the blood-sucking bug *Triatoma pallidipennis*. See Example 1.

Thus, this invention relates to a purified and isolated protein which inhibits collagen-induced aggregation of human platelets. The protein is isolatable from *Triatoma pallidipennis*. It also relates to pharmaceutical compositions containing a protein and methods of using the latter for treating thrombotic lesions or for preventing reocclusion after treatment of myocardial infarction, and for treatment of progression of metastasis; *inter alia*.

Thus, this invention provides a valuable pharmacologically active substance, e.g., a new protein which specifically inhibits the collagen-induced platelet aggregation with a high specific activity; a new protein which specifically interferes with the platelet-collagen interaction without causing release of intraplatelet constituents, e.g., ATP, which have undesirable side effects by themselves; a new protein for pharmaceutical use in treating atherosclerotic and thrombotic diseases or in preventing reocclusion after treatment of myocardial infarction; a new protein which interferes with tumor cell-collagen interaction and which can be used, e.g., to prevent tumor cell metastasis.

An investigation of the characteristics and properties of the inhibitor yielded the following results:
1) The inhibitor is not a fibrinogen-receptor antagonist, because experiments with increasing fibrinogen concentrations showed no influence on the inhibitory activity. See Example 3.
2) It is not a thromboxane-antagonist, because it prevents the collagen-induced aggregation of platelets pretreated with aspirin but not the U46619 (a thromboxane mimeticum) induced aggregation. See Example 4.
3) It is probably not an inhibitor of the protein kinase C mediated signal transduction pathway, because the aggregation induced by phorbolesters (phorbol-12-myristate-13-acetate) is not inhibited. See Example 4.
4) It inhibits the release-reaction of collagen-treated platelets. See Example 5.
5) It does not inhibit the platelet aggregation induced by thrombin or ADP. See Example 6.
6) The inhibitor does not react with collagen. While preincubation of the inhibitor with collagen does not yield an increased inhibitory activity, a prolonged incubation of the platelets with the inhibitor leads to a higher inhibitory potency. See Example 7.
7) The inhibition of platelet aggregation becomes reversible by the addition of a large amount of collagen. See Example 7.
8) Protease inhibitors do not have measurable influence on the activity. See Example 8.
9) The inhibitory activity is higher in the presence of Mg²⁺-ions. See Example 9.
10) The inhibitor prevents the adhesion of platelets to a collagen matrix in a dose-dependent manner. See Example 10.
   These results strongly imply that the inhibitor is a collagen receptor antagonist of high specific activity, e.g. IC₅₀ = 2.5 µg per ml of the "Superose"-pool fraction described below (based on partially purified inhibitor) and IC₅₀ = 50 nmol/l of the highly purified protein (purified according to the consecutive steps described in the Examples 2 and 15).
11) The inhibitor was incubated with trypsin bound to a Sepharose-matrix. The inhibitory activity was totally lost by proteolytic digestion. See Example 11, showing that the inhibitor is a protein.
12) The inhibitor is not cleavable by collagenase. See Example 12.
13) According to gel filtration chromatography in the presence of 150 mM NaCl, the inhibitor has a molecular weight of 20 kDa ± 5 kDalton, i.e., about 20 kDalton. See Example13. The value of the non-glycosylated protein (See Seq Id No 1) calculated for the cDNA sequence is 18,923 Dalton.
14) The inhibitor prevents the adhesion of highly metastatic tumor cells (MTLn3) to collagen in a dosedependent manner. See Example 14.

The inhibitor of this invention does not bind to (or react with) collagen, does bind to platelets, and does not cause flocculation of collagen.

The collagen inhibitor of this invention can be routinely isolated from saliva of the blood-sucking bug *Triatoma pallidipennis*, e.g., as described in the examples herein. Conventional saliva harvesting methods are fully applicable to provide the starting material saliva. The bug *Triatoma pallidipennis* is prevalent and thus readily available in Central and South America. It is known as a vector for *Trypanosoma cruzi*.

In another aspect of this invention, there are provided DNA sequences, vectors containig theses sequences, cells containing said vectors, methods of recombinantly producing proteins and antibodies to the proteins of this invention. Also provided are isolated and/or recombinant DNA sequences (e.g., genomic or cDNA) coding for a protein (e.g., naturally occurring) which inhibits collagen-induced aggregation of human platelets. In a still further aspect, the invention provides recombinantly produced proteins of this invention, e.g., having the sequences disclosed herein.

By the term "isolated" is meant that the inhibitor of this invention or other entity is present in a form separated from (purified from) components with which it is naturally combined or with which it is produced recombinantly or synthetically.

All degrees of such isolation or purification are included generically. Preferred are degrees of isolation or purification whereby the inhibitor is useful for pharmaceutical purposes. For example, such degrees of isolation (e.g., activities or purities) can be routinely achieved by chromatographic techniques such as those used in the examples. Further purifications, e. g., to homogeneity, can be routinely achieved using conventional methods, such as those described in the following texts:
Methods of Enzymology, Volume **182**, Guide to Protein Purification, ed. Murray P. DEUTSCHER, Academic Press 1990;
Protein Purification Applications - A Practical Approach. ed. E.L.V. Harris and S. ANGEL, IRL-Press 1990;
Protein Purification, Principles and Practice, Robert SCOPES, Springer-Verlag 1982;
   and
Protein Purification, Principles, High Resolution Methods and Applications, ed. J.-C. JANSON and L. RYDEN, VCH publishers 1989.

Purity can be determined by any one of a number of routine methods, e.g., SDS polyacrylamide gel electrophoresis, analytical HPLC, etc. Purified inhibitor can be used to determine the amino acid sequence of the protein according to methods fully routine to one of ordinary skill in the art. HEWICK, R.M. et al. (1981) J. Biol. Chem. **256**, 7990-7997.

The protein of this invention includes not only the protein isolated from the exemplified species of insect, but also any other organism which may contain said inhibitor. In addition, the inhibitor of this invention includes inhibitors having related structure, e.g., a collagen-induced platelet aggregation inhibitor isolated from another organism which has a substantially similar amino acid sequence.

Since this protein is isolated from a biting insect, and its natural utility is apparently to keep a bite wound in a host unobstructed by blood clots for an extended period of time in order to effect the intake of a blood meal, it is quite likely that other such collagen-induced platelet aggregation inhibitors will be found in the saliva of other blood-sucking organisms, especially insects, e.g., in other cone-nosed Reduviid bugs of the subfamily Triatominae, such as *Triatoma infestans*, *T*. *dimidiata*, *T. maculata, Rhodnius prolixus*, *Panstrongylus megistus* and *P. infestans*.

Proteins of this invention include monomeric, single chain molecular forms, i.e., those not covalently or noncovalently bonded to other polypeptide chains. This invention also encompasses other molecular forms of the protein, e.g., dimers or other oligomers, tertiary structures formed with other polypeptides, fragments of the protein, etc. Both glycosylated and unglycosylated forms are included, both forms being routinely preparable by expression from, e.g., mammalian (glycosylated) or bacterial cells (unglycosyltated), respectively.

The amino acid sequence of the inhibitor of the present invention can be used to determine the sequence of suitable DNA probes, which can be used for finding new inhibitors, e.g., in other species. Such probes can be routinely synthesized, e.g., using automated DNA synthesizers, and screening of genomic or cDNA libraries is similarly routine for one of ordinary skill in the art. (See international Publication WO 90/07861 dated 26 July, 1990)

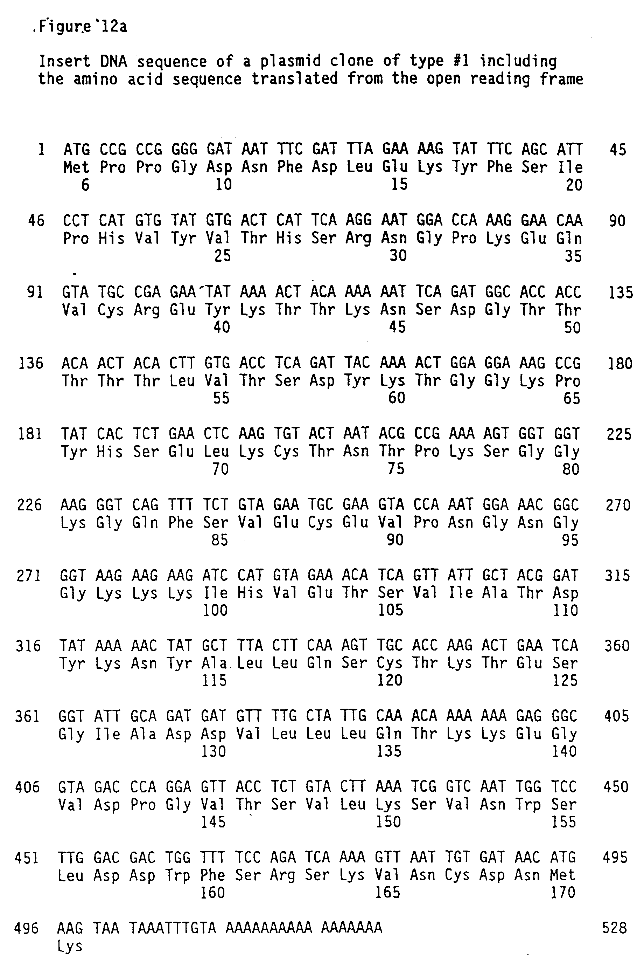

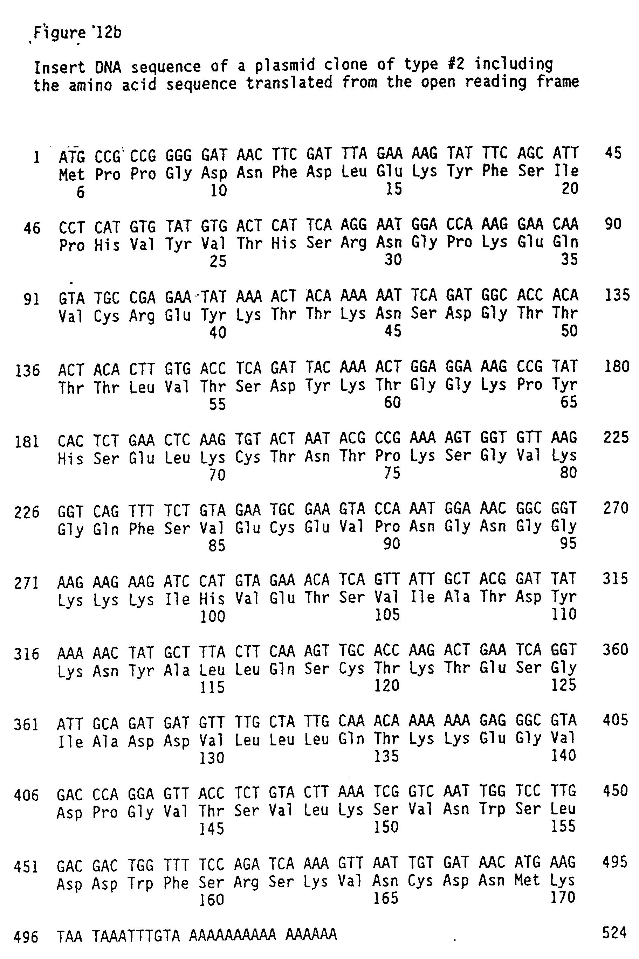

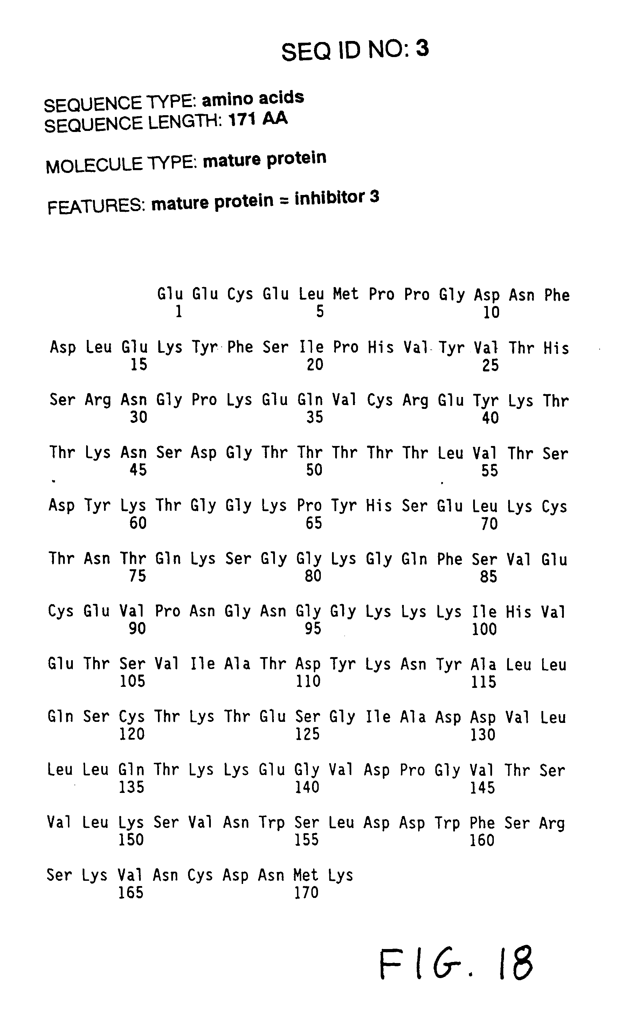

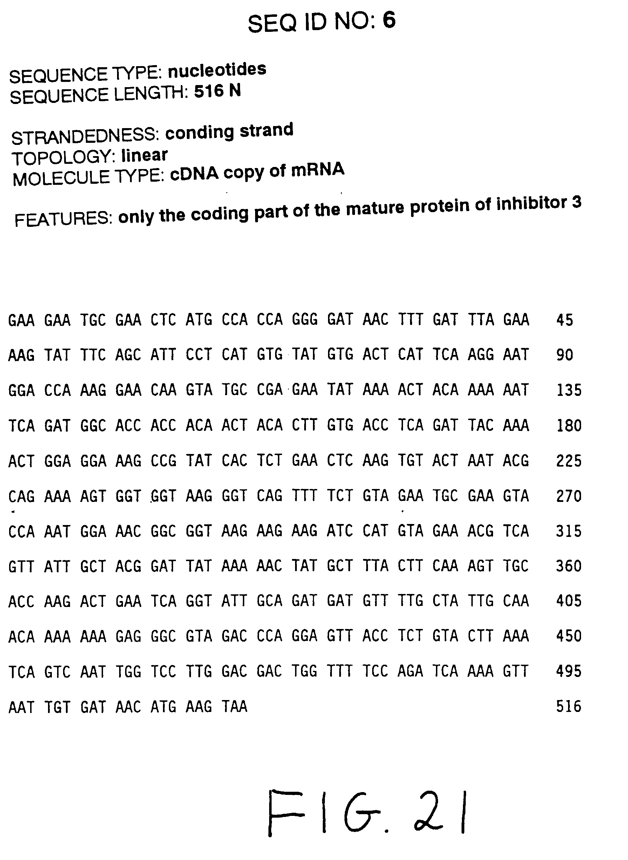

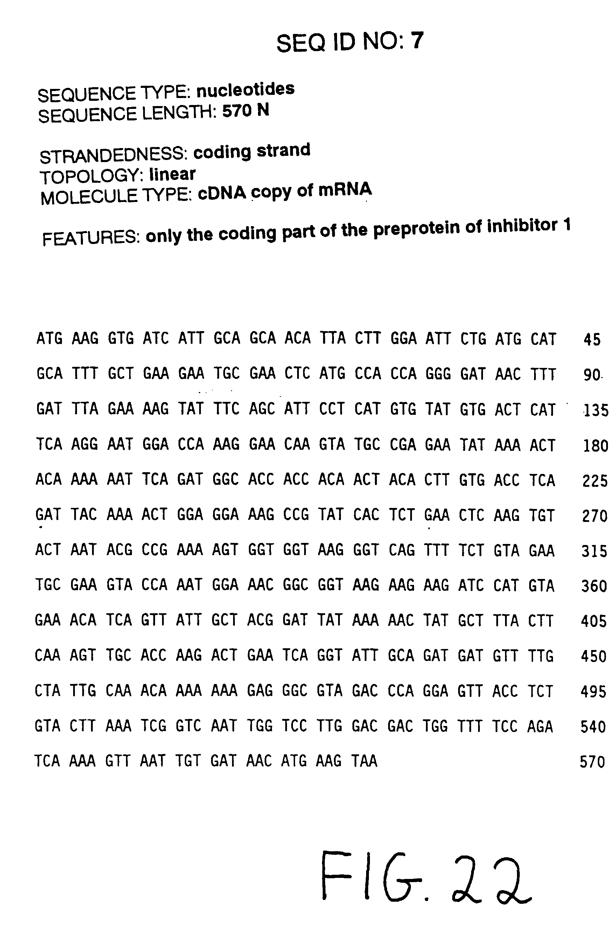

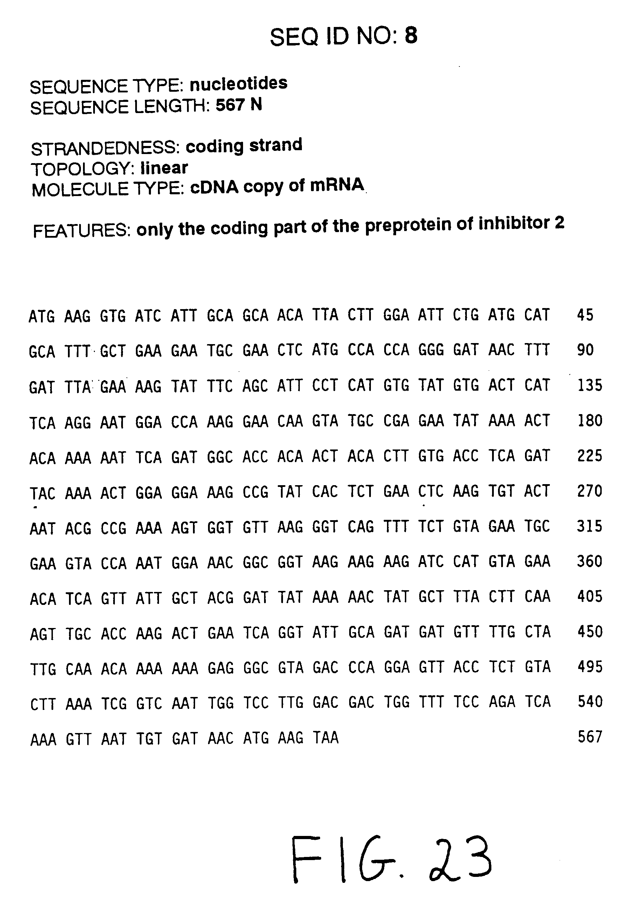

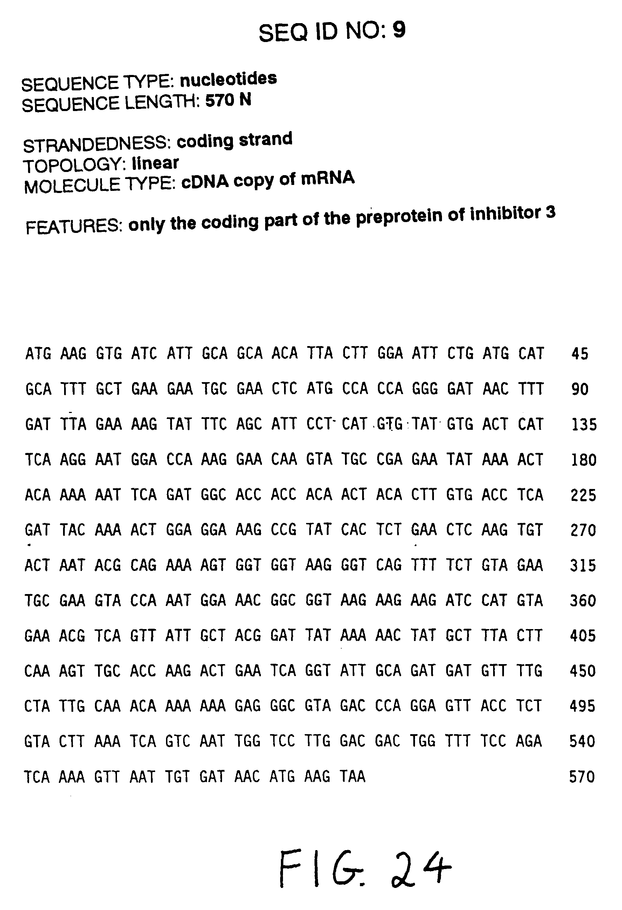

For example, the invention relates to DNA sequences as disclosed in Figures 12a - c, 13a and b and 22 - 24. Still further, the invention relates to DNA sequences coding for muteins as defined above. The sequence for the -18 to +5 region in Figure 18, 21 and 24 are deduced from the corresponding full length cDNA sequences of inhibitors 1 and 2.

Therefore, the present invention also includes the DNA sequence corresponding (coding for) to both the natural DNA sequence (gene) for the inhibitor, when isolated from the natural environment, e.g., in solution or on a vector, as well as muteins thereof, either naturally occuring, e.g., in other species, in isolated form or synthetic, e.g., as produced by site-directed mutagenesis. Methods of screening genetic libraries of various species with a suitable probe are conventional in the art. Methods for producing muteins are also routine and conventional for one of ordinary skill in the art, as are screening methods for testing the efficacy of such new proteins, e. g., as decribed herein.

Suitable muteins, either synthetic or naturally occuring (in isolated form), are those having at least a fraction, e.g., at least 5%, preferably at least 50%, most preferably at least 90% of the biological activity, e.g., collagen-induced platelet aggregation inhibition, of naturally occuring, isolated *T. pallidipennis* inhibitor as described herein.

Suitable muteins will differ from the natural proteins in any modification possible, including deletion, addition and/or substitution of one or more amino acids as long as substantial biological acitivity is retained; preferably the biological acitivity is substantially unaffected. Such muteins are equivalents of the natural proteins. Similarly, equivalents of the DNA sequences disclosed herein include allelic variants, sequences coding for the equivalent muteins discussed herein and DNA sequences which are homologous therewith.

Furthermore, the invention includes fragments of the thrombolytic polypeptide, e.g., having similar functions or isolated subfunctions, e.g., isolated epitopes, acitive sites, fibrin and/or fibrinogen binding regions, etc. Single chain forms of the inhibitor are preferred.

The invention also relates to antibodies and antibody-producing cell lines, in particular monoclonal antibodies and cell lines, which can routinely be produced using the purified proteins of this invention, e.g., according to the highly conventional KÖHLER and MILSTEIN method involving conventionally immunizing mice with the purified protein of the invention as immunogen. The invention also relates to fragments of said antibodies, e.g. antibody fragments containing a domain which bindes to an epitope on the inhibitor protein and to synthetic binding domains, e.g., mimotopes, specifically recognizing domains on the proteins of this invention

The inhibitor of collagen-induced platelet aggregation of human (and other mammalian) platelets of this invention can be used as an antiatherosclerotic and antithrombotic agent in mammals, including humans, e.g., to treat atherosclerotic/thrombotic lesions, for example, due to rupture of atherosclerotic plaques, those derived from angioplasty (PTA/PTCA) or those due to perturbation or removal of endothelium, e.g., in sepsis or transplants. It can also be used to treat unstable angia and/or to prevent reocclusion after treatment of myocardial infarction. Details of such uses, e.g., dosage ranges, regimes of administration (preferably, oral or parenteral), etc., can be routinely determined, e.g., by analogy to and/or routine comparison with other antithrombotic agents such as t-PA, streptokinase, or other platelet-aggregation inhibitors such as Iloprost, etc.

The inhibitor of this invention can also be used to prevent metastasis of tumor cells by blocking their passage through the connective tissue. It is applicable to prevent metastasis of all invasive tumors, e.g., melanoma. The following is offered without wishing to be bound by theory. During metastasis, the tumor cells have to penetrate through the base membrane and interstitial matrix. Both matrices are rich in various collagen types. The dissemination of the tumor cells requires interaction with these proteins. Evidence for the role of the collagen receptor (VL A 2) in this interaction is provided by, e.g., CHAN et al. (1990, Science 2, 1600-1602) who cloned the VLA 2 positive tumor cells which formed substantially more metastatic tumor colonies. KRAMER and MARKS (J. Biol. Chem. 1989, **264**, 4684-4688) were able to block the attachment of human melanoma cells to collagen by an antibody to VLA 2. See also: PA US 73234708-A "Monoclonal antibody against platelets - which inhibit platelet reaction with collagen and are used for detecting and treating cancer", U.S. Dept. Health and Human Services. The inhibitor of this invention, being a collagen receptor antagonist prevents the interaction of tumor cells with the surrounding matrix and thus inhibits metastasis.

It is also employable as a standard for determining the effectiveness of new inhibitors which can be developed, e.g., by modification of the structure of the present inhibitor through standard mutagenesis, directed mutagenesis, e.g., deletions and/or insertions of sequences, protein modifications, etc. The inhibitor of this invention can also be used as a standard antithrombotic in screening procedures which test for the effectiveness of various compounds as an antithrombotic, or as a standard for determining the effectiveness of compounds which block the effects of such collagen-induced platelet aggregation, e.g., in patients suffering from clotting deficiencies.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

### DESCRIPTION OF THE DRAWINGS

Various other objects, features and attendant advantages of the invention will be more fully appreciated as the same becomes better understood when considered in conjunction with the accompanying drawing, in which like reference characters designate the same or similar parts throughout the several views, and wherein:
- Figure 1: shows the dose-dependent inhibition of human platelet aggegation by the saliva of *Triatoma*;
- Figure 2: shows the does-dependent inhibition of human platelet aggregation by the "Superose"-pool of the saliva of *Triatoma*;
- Figure 3: shows the gel filtration pattern on "Superose 12";
- Figure 4: shows the independence of aggregation inhibition on fibrinogen concentrations;
- Figure 5: shows the prevention of the collagen-induced aggregation of platelets pretreated with aspirin;
- Figure 6: shows no reaction of the inhibitor with collagen;
- Figure 7: shows the proteolytic digestion of the inhibitor;
- Figure 8: shows the stability of the inhibitor against collagenase;
- Figure 9: shows the determination of the molecular weight of the inhibitor;
- Figure 10: shows the purification fo the inhibitor to homogeneity;
- Figure 11: shows the sizes of the PCR products;
- Figure 12a: shows the DNA sequence of the subcloned PCR product of type 1 and the deduced amino acid sequence;
- Figure 12b: shows the DNA sequence of the subcloned PCR product of type 2 and the deduced amino acid sequence;
- Figure 12c: shows the DNA sequnce of the subcloned PCR product of type 3 and the deduced amino acid sequence;
- Figure 13a: shows the complete DNA sequence of the cloned cDNA of inhibitor 1 and the corresponding amino acid sequence;
- Figure 13b: shows the complete DNA sequence of the cloned cDNA of inhibitor 2 and the corresponding amino acid sequence;
- Figure 14: shows the expression plasmid including the DNA coding for the inhibitor;
- Figure 15: shows the molecular weight of the mature recombinant protein detected by antibodies which specifically recognize the mature protein;

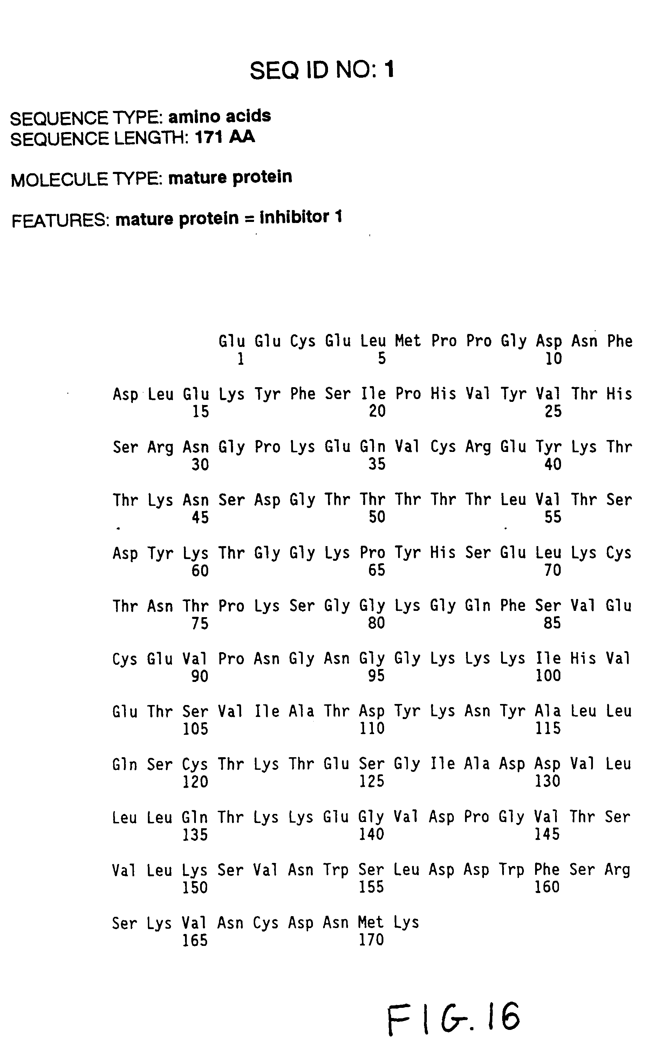

- Figure 16: shows the mature protein sequence of inhibitor-1;

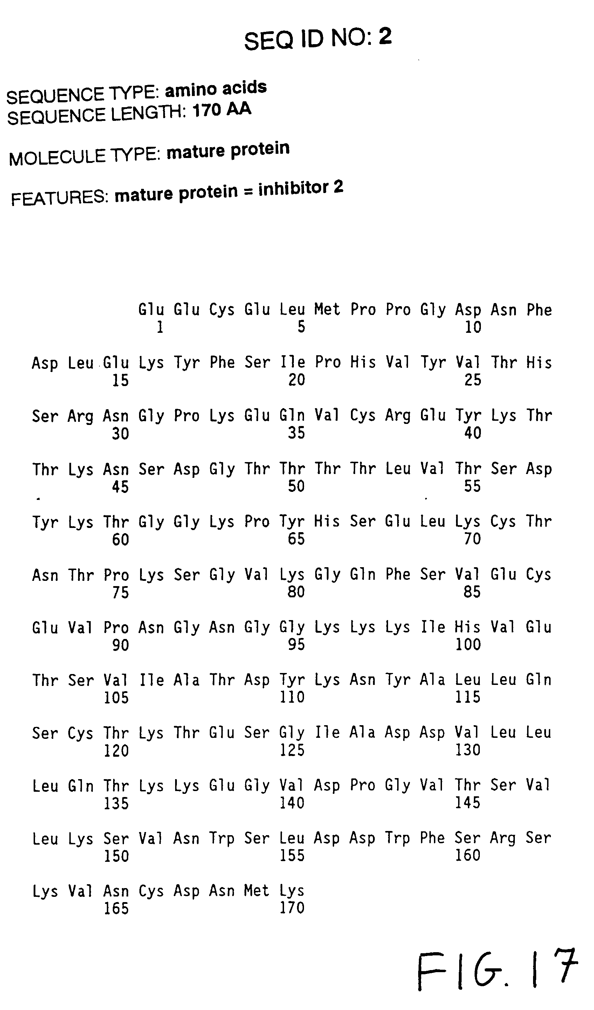

- Figure 17: shows the mature protein sequence of inhibitor-2;
- Figure 18: shows the mature protein sequence of inhibitor-3

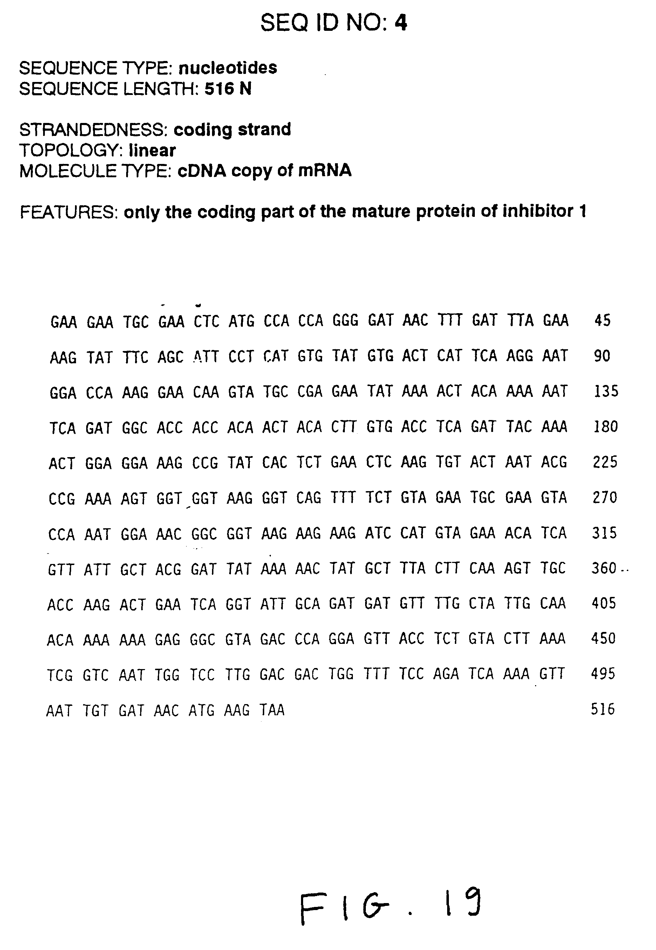

- Figure 19: shows the DNA coding sequence of the mature protein of inhibitor-1;

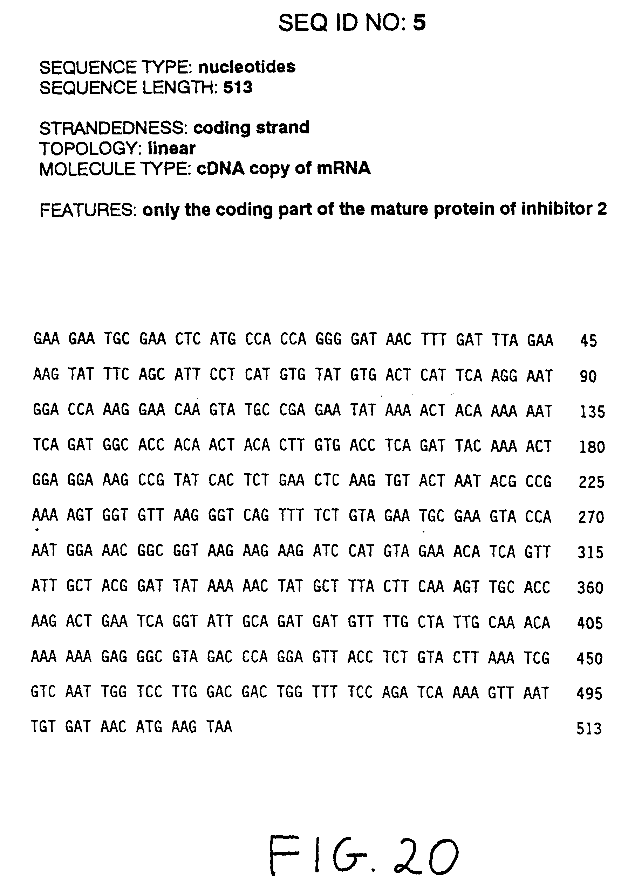

- Figure 20: shows the DNA coding sequence of the mature protein of inhibitor-2;
- Figure 21: shows the DNA coding sequence of the mature protein of inhibitor-3;
- Figure 22: shows the DNA coding sequence of the pre-protein of inhibitor-1;
- Figure 23: shows the DNA coding sequence of the pre-protein of inhibitor-2;
- Figure 24: shows the DNA coding sequence of the pre-protein of inhibitor-3;
- Figure 25: shows the flow diagram of finding the cDNA of the invention and

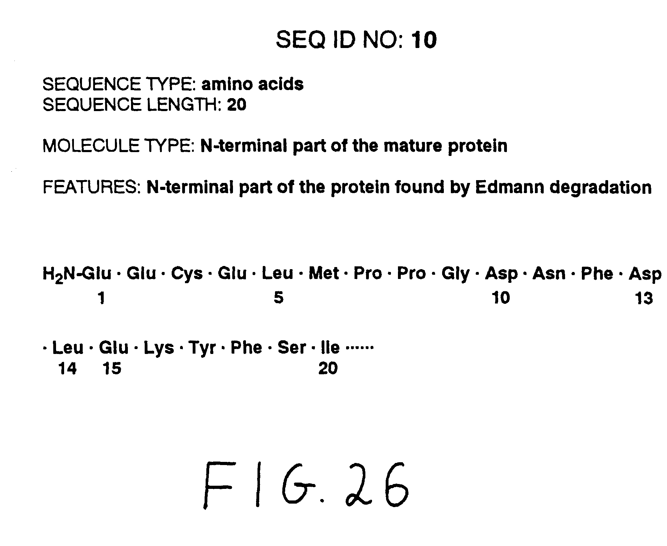

- Figure 26: shows the N-terminal amino acids of the protein of the invention in Seq Id No. 10.

### List of the Sequence Identifier:

Sequence Identifier 1 shows the mature protein sequence of inhibitor-1;
Sequence Identifier 2 shows the mature protein sequence of inhibitor-2;
Sequence Identifier 3 shows the mature protein sequence of inhibitor-3
Sequence Identifier 4 shows the DNA coding sequence of the mature protein of inhibitor-1;
Sequence Identifier 5 shows the DNA coding sequence of the mature protein of inhibitor-2;
Sequence Identifier 6 shows the DNA coding sequence of the mature protein of inhibitor-3;
Sequence Identifier 7 shows the DNA coding sequence of the preprotein of inhibitor-1'
Sequence Identifier 8 shows the DNA coding sequence of the preprotein of inhibitor-2;
Sequence Identifier 9 shows the DNA coding sequence of the preprotein of inhibitor-3; and
Sequence Identifer 10 shows the N-terminal amino acids of the mature protein.

In the foregoing and in the following examples, all temperatures are set forth uncorrected in degrees Celsius and unless otherwise indicated, all parts and percentages are by weight. The entire disclosures of all applications, patents and publications, cited above and below, are hereby incorporated by reference.

### Examples

### Acitivity of the Inventive protein (Example 1)

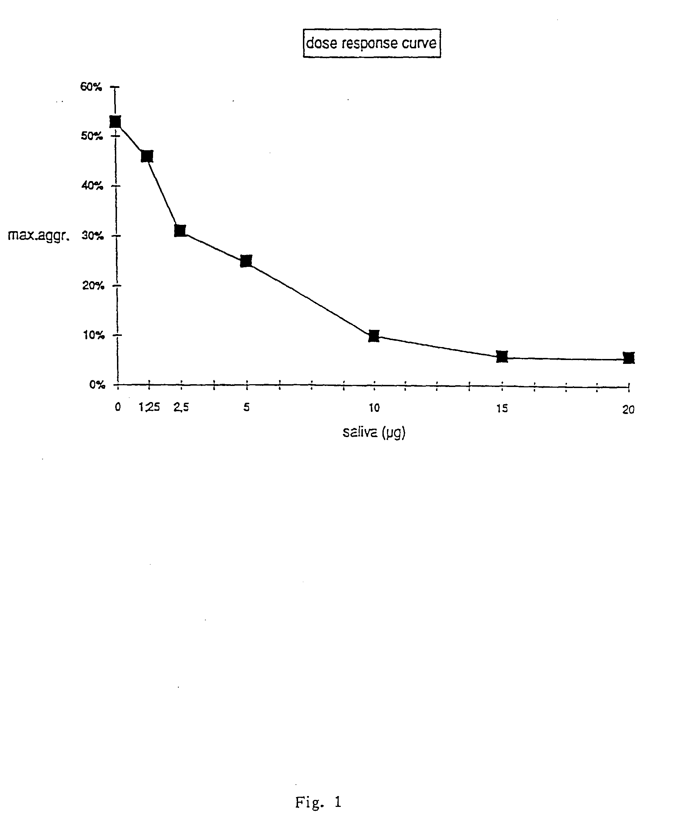

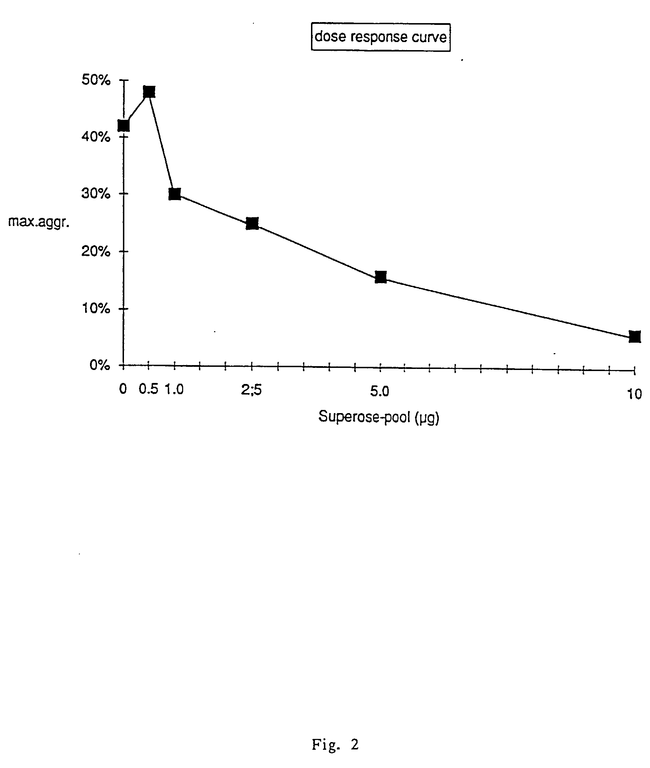

**The aggregation formed by human platelets in the presence of collagen is inhibited, when saliva of *Triatoma pallidipennis* or purified protein is added. The inhibition correlates with the concentration of saliva or protein.**
The bugs are stimulated to eject their saliva onto a siliconized glassplate by mechanical stimulation of the probszis. The ejected material is collected using drawn-out siliconized pasteur pipettes. 500 µl platelet rich plasma (300,000 platelets/µl) is incubated with different amounts of saliva (1.25-20 µg protein in 20 µl) or with different amounts of "Superose"-pool (0.5-10 µg protein in 200 µl) from Example 2 at 37 °C in an aggregometer. After 1 min, 1µg of collagen is added and the increase in light transmission (aggregation) is monitored. See Figures 1 and 2.

### Purification of the protein (Example 2)

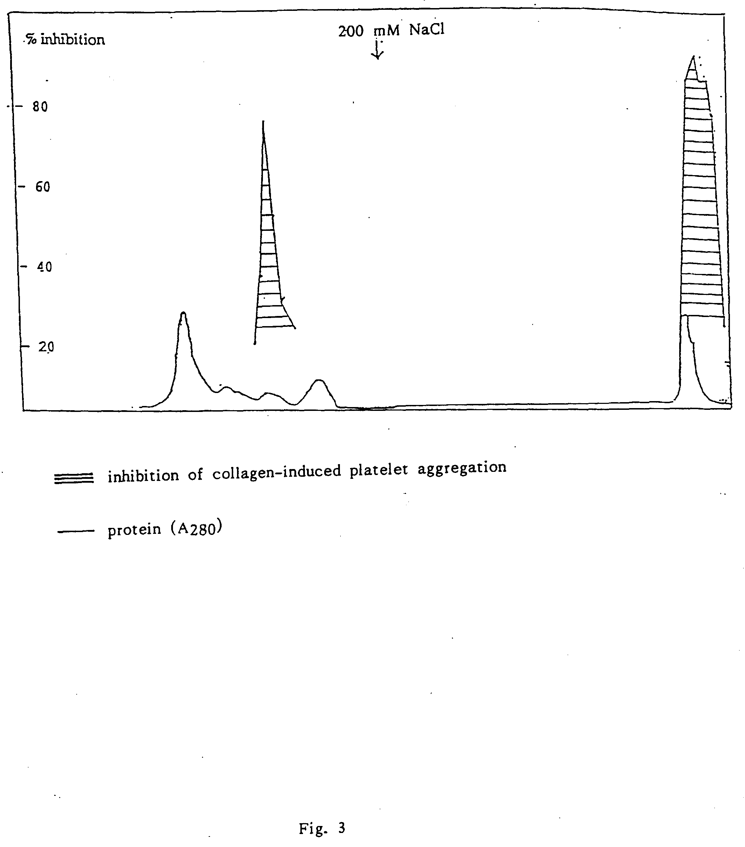

**The important step to purify the protein is the use of the gel filtration using** ^{**"**}**Superose 12".** 2 ml (5 mg protein) of saliva is chromatographed over a "Superose 12" HR 16/50 chromatography column (Pharmacia) in 10 mM Tris/HCl, pH 7.4; 0.0001% "Pluronic F68". Thereafter, the inhibitor is eluted with 10 mM Tris/HCl, pH 7.4; 0.0001% "Pluronic F68", 200 mM NaCl. See Figure 3. The inhibitor pool contains 25 µg/ml protein. 5 µg of protein shows a 70% inhibition of aggregation. The aggregation of platelets without inhibitor is defined as 100% aggregation and 0% inhibition. Accordingly the other data are calculated.

### The inhibiton is independent of fibirinogen (Example 3)

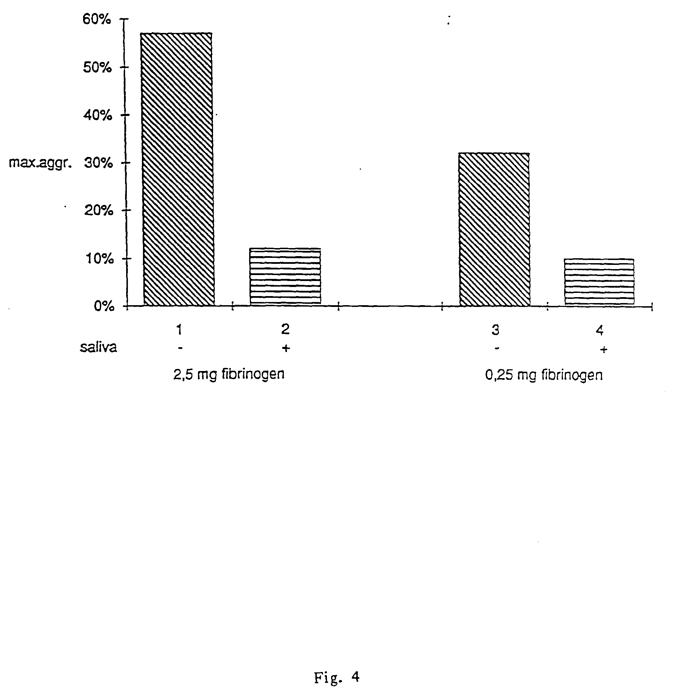

**The inhibitory activity which is shown by the protein of the invention is independent of the concentration of added fibrinogen.** 500 µl of gel filtrated platelets is combined with fibrinogen and 50 µg saliva. After incubation for 1 min at 37 °C, 1 µg of collagen is added and the aggregation is monitored. The the values represended by the bars 2 and 4 (with saliva) do no show significant differences wherein the values represended by the bars 1 and 3 (without saliva) correlate with the concentration of fibrinogen. See Figure 4.

### Test to detect the mechanism of the platelet aggregation induced by the inventive proteins (Example 4)

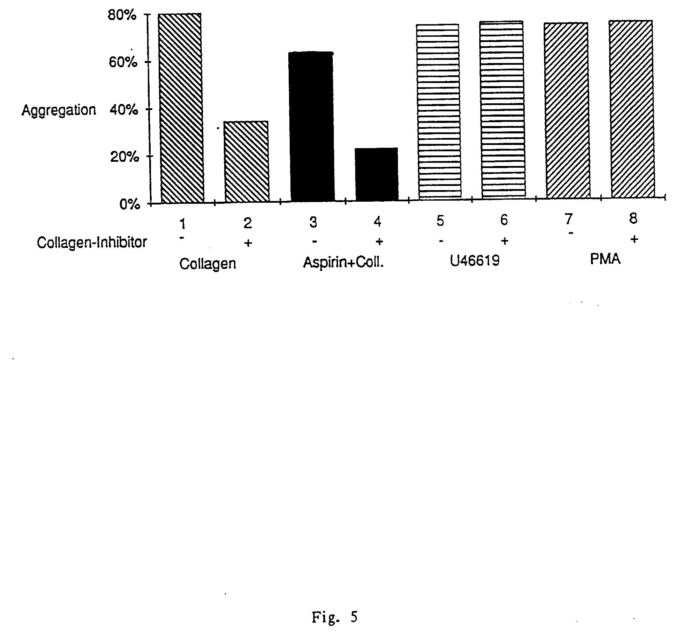

**In order to study the mechanism of the protein of the invention some standard compounds are added to a test system in which alternatively the inventive protein or buffer is present. The activity of the inventive protein is not significantly altered when the compound aspirin is added (bar 4). Wherein, the effect of the compounds U46619 and PMA cannot be influenced by the protein of the invention. Therefore, the protein of the invention is no thromboxane antagonist and probably no inhibitor of protein kinase C.**
**Bars 1 and 2:** 500µl of platelet-rich plasma (300,000 platelets/µl) is incubated with the protein of the invention (bar 2) (200 µl "Superose"-pool) or with buffer (bar 1) respectively at 37 °C for 1 min. Then 1 µg collagen is added and the aggregation is monitored in an aggrigometer.
**Bars 3 and 4:** 500µl of platelet-rich plasma is incubated with 1 mM of aspirin for 20 min at room temperature. Thereafter the protein of the invention (bar 4) or buffer (bar 3) is added. After an incubation period of 1 min at 37 °C, 1 µg of collagen is added and the aggregation is monitored.
**Bars 5 and 6:** 500µl of platelet-rich plasma is incubated with the protein of the invention (bar 6) or buffer (bar 5) respectively for 1 min at 37 °C. Then U46619 (1 µM) is added and the aggregation is monitored.
**Bars 7 and 8:** 500µl of platelet-rich plasma is incubated with the protein of the invention (bar 8) or buffer (7) respectively at 37 °C in an aggregometer. After 1 min, 10 ng of PMA (phorbol-12-myristate-13-acetate) is added and the aggregation is monitored.
**Results** are shown in Figure 5.

### Platelet-release reaction (ATP measurement) (Example 5)

**When platelet and collagen are present, the protein of the invention can inhibit the activation of the platelets. ATP is used as an indicator for** **activation.** 500 µl of platelet-rich-plasma is incubated with 200 µl of protein of the invention (Superose-pool) or H₂O respectively at 37 °C for 1 min. Then 1 µg of collagen is added. The aggregation is monitored for 10 min. Thereafter, 200 µl of the suspension is combined with 250 µl Hepes buffer pH 7.4, 100 mM luciferin and 5 µg/ml luciferase. Then the luminescence is measured.

The total ATP content of the platelets is determined after lysis of the platelets with "Nonidet P40".

| amount of inventive protein added | max. aggr. | released ATP % of total ATP content |
|---|---|---|
| ----- | 68% | 49% |
| 15 µl (= 5 µg protein) | 49% | 15% |
| 30 µl (=10 µg protein) | 18% | 2% |

### Inhibition of platelet aggregation induced by different substances (Example 6)

**The protein of the invention is specific for collagen-induced platelet aggregation.** 500 µl of filtrated platelets (300,000 platelets/µl) are incubated with the protein of the invention for 1 min at 37 °C. Then the aggregation is induced with collagen (2 µg/ml), thrombin (0.06 U/ml) or ADP (1 · 10⁻⁵ M) respectively and the aggregation is monitored.

| | maximal aggregation | | |
|---|---|---|---|
| | collagen | thrombin | ADP |
| **control** | 64% | 75% | 57% |
| partially purified **protein** (200 µl) "Superose"-pool | 23% | 75% | 44% |

### Inhibition of collagen-induced aggregation (Example 7)

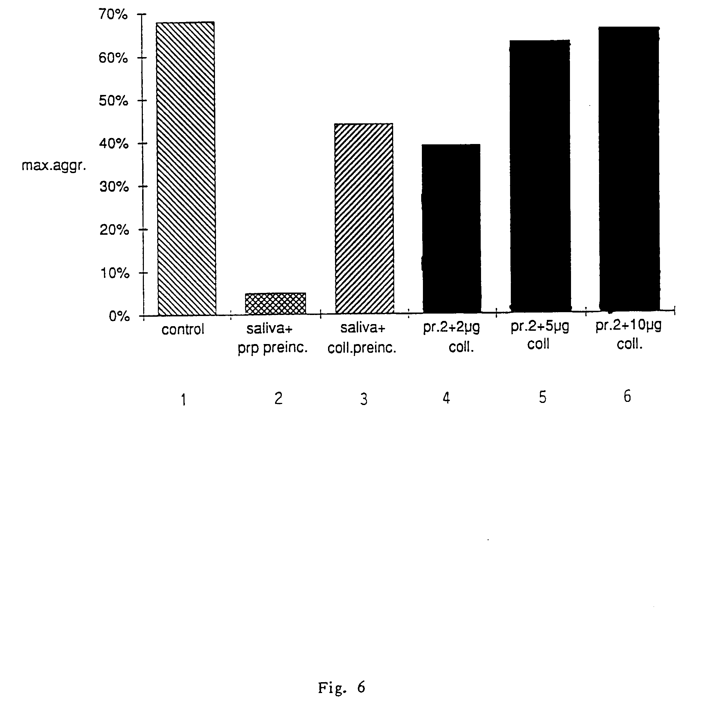

**The protein of the invention does not react with collagen. The inhibition of collagen-induced platelet aggregation in presence of the protein can be neutralized by a surplus of additionally added collagen**. The collagen-induced aggregation (2 µg/ml) of 500 µl of platelet-rich plasma is measured with the following modifications:
1: control, without inhibitor;
2: protein of the invention (100 µg of saliva) 10 min preincubated with human platelets prior to addition of collagen;
3: inhibitor (100 µg of saliva) 10 min preincubated with collagen prior addition of platelet-rich-plasma
4,5,6: after measurement of the aggregation, 2, 5 and 10 µg of collagen, respectively, is added to probe No. 2 and aggregation is measured again. See Figure 6.

### Impact of protease inhibitors on the inhibitory activity (Example 8)

**The protein of the invention is no protease.** Protease inhibitors (2 mM PMSF, 2mM leupeptin, 2mM aprotenin) or buffers is incubated for 15 min with the "Superose"-pool (200 µl). Then platelet-rich-plasma is added and the aggregation is started with collagen (2 µg/ml).

| | maximal aggregation |
|---|---|
| control (without inventive protein) | 86% |
| + "Superose"-pool | 52% |
| +"Superose"-pool + protease inhibitors | 48% |

### The inhibition is dependent on the presence of Mg²⁺ (Example 9)

**The cation Mg**^{**2+**} **increases the inhibation of platelet aggregation by the protein of the invention. Wherein the cation Ca**^{**2+**} **has no significant influence on the inhibition of the platelet aggregation.** 500 µl of platelet-rich plasma is combined with 2 mM Mg²⁺ or Ca²⁺ and 40 µg saliva or buffer. After incubation for 1 min at 37 °C, 1 µg of collagen is added and the aggregation is monitored.

| Additives | maximal aggregation |
|---|---|
| ----- | 77% |
| saliva | 33% |
| 2 mM Mg²⁺ | 75% |
| saliva + 2 mM Mg²⁺ | 19% |
| 2 mM Ca²⁺ | 63% |
| saliva + 2 mM Ca²⁺ | 39% |

### Platelets incubated with the protein of the invention show decreased adhesion to collagen. (Example 10)

**When platelets are incubated with the inventive protein, they partially loose their capability to bind collagen.** A 96 well plate is coated with collagen (type I at 4 °C overnight). 1 · 10⁷ platelets per well are incubated with different amounts of inhibitor and 2 mM Mg²⁺ for 20 min at 37 °C with agitation. They are washed with PBS and the adherent platelets are fixed with 2.5% glutardialdehyde for 2 h at 37 °C. Then the platelets are removed from the well and counted under a microscope.

| protein* | adherent platelets per mm² |
|---|---|
| ---- | 13500 |
| 10 µl | 12000 |
| 50 µl | 6500 |

| | |
|---|---|
| * protein = "Superose"-pool concentrated to 0.5 mg protein/ml | |

### Incubation of the inhibitor with trypsin-Sepharose (Example 11)

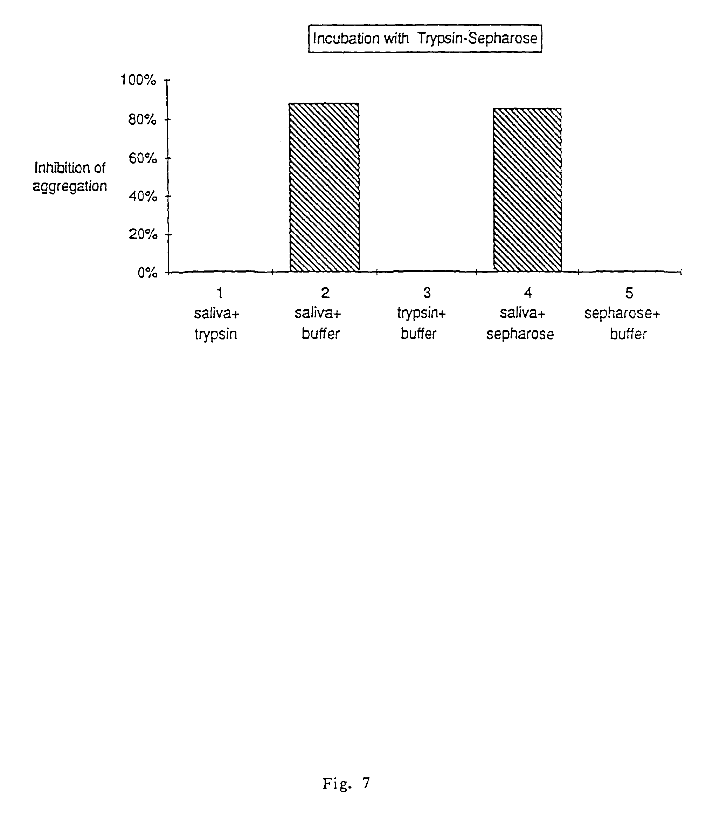

**The protein of the invention is digested by trypsin bound to Sepharose. The digestion of the inventive protein results in a complete loss of the acitivity of the inventive protein.** 200 µg of saliva is combined with trypsin bound to Sepharose or with buffer or with Sepharose respectively and trypsin-Sepharose is combined with buffer. All batches contain 150 mM of NaCl to prevent nonspecific adsorption to the matrix. After incubation overnight at room temperature with aggitation, the batches are centrifuged and the supernatants are added to an aggregation assay (500 µl of platelet-rich-plasma, 1 µg of collagen). The proteolytic digestion is monitored on a SDS-polyacrylamide gel. See Figure 7.

### Incubation of the inhibitor with collagenase-Sepharose (Example 12)

**The protein of the invention is not cleavable by a collagenase.** Collagenase (*Clostridium histolyticum)* is bound to Sepharose and used in the following batches:
1. 100 µl of collagenase-Sepharose + 60 µl of protein of the invention + 140 µl of H₂O + 10 µl buffer.
2. 100 µl of 150 mM NaCl, 50 mM Tris/HCl pH 7.4 + 60 µl of protein of the invention + 140 µl of H₂O + 10 µl of buffer.
3. 100 µl of collagenase-Sepharose + 200 µl of H₂O + 10 µl of buffer.
   As a control, bovine serum albumin is coupled to Sepharose and is used in in the following batches:
4. 100 µl of BSA-Sepharose + 60 µl of protein of the invention + 140 µl of H₂O + 10 µl of buffer.
5. 100 µl of BSA-Sepharose + 200 µl of H₂O + 10 µl of buffer.
protein: "Superose"-pool
buffer: 140 mM Tris/HCl, pH 7.4, 100 mM CaCl₂.

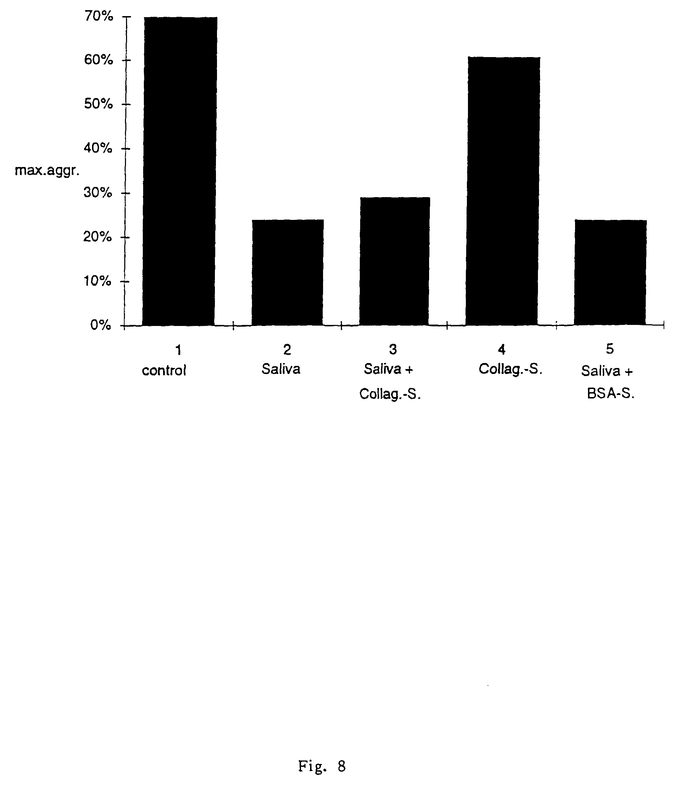

The batches are centrifuged and 200 µl of each supernatant is incubated with 500 µl of platelet-rich plasma for 1 min at 37 °C. Then 1 µg collagen is added and the aggregation is monitored. The activity of the collagenase-Sepharose is monitored by incubating it with collagen and running a SDS-polyacrylamide gel electrophoresis. See Figure 8.

### Molecular mass determination for the inhibitor by gel filtration (Example 13)

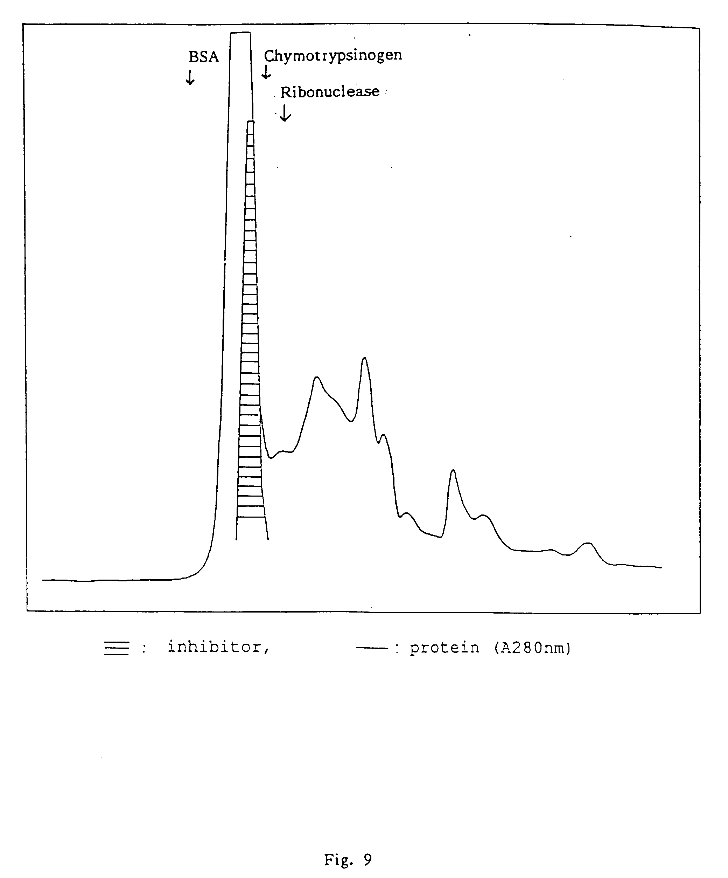

**The purified protein of the invention shows a molecular weight of 20,000 ± 5,000 Dalton measured by a Superose 12 column filtration. The** "Superose"-pool from 2 ml of saliva (see Example 2) is chromatographed over a "Superose 12" HR16/50 column in Tris/HCl pH 7.4, 150 mM NaCl, 0.0001% "Pluronic" F68. Bovine serum albumin (MW 67kDa), chymotrypsinogen (MW 25 kDa) ribonuclease (MW 14 kDa) are used as molecular weight makers. See Figure 9.

### The adhesion of tumor cells to collagen is decreased in the presence of the protein of the invention (Example 14)

**The protein of the invention inhibits the adhesion of tumor cells to a collagen matrix. Therefore, migrating tumor cells can be prevent partially or completely from settling down in organs or blood vessels, when the protein of the invention is within the blood or the plasma of the patient.** MTLn3 cells (rat mammary tumor cells) are labelled with ⁵¹Cr. A well plate is coated with collagen (type III) at 4 °C overnight. 2 · 10⁴ labelled cells in 500 µl DMEM F12 medium, 20 mM Hepes, 1 mM bicarbonate, 1% BSA are first incubated with 0, 2, 5 or10 µl protein of the invention ("Superose Pool", 0.5 mg protein/ml) respectively for 10 min at 37 °C. Then this suspension is transferred to a collagen-coated well and incubated for 2 h at 37 °C. Thereafter, the wells are washed and the adherent cells are removed with 1 M NaOH. The radioactivity of the adherent cells are counted.

| **amount of the inhibitor added µl** | **cell attachment (cpm)** |
|---|---|
| 0 | 2215 |
| 2 | 2071 |
| 5 | 1608 |
| 10 | 1081 |

### Purification of the inhibitor to homogeneity (Example 15)

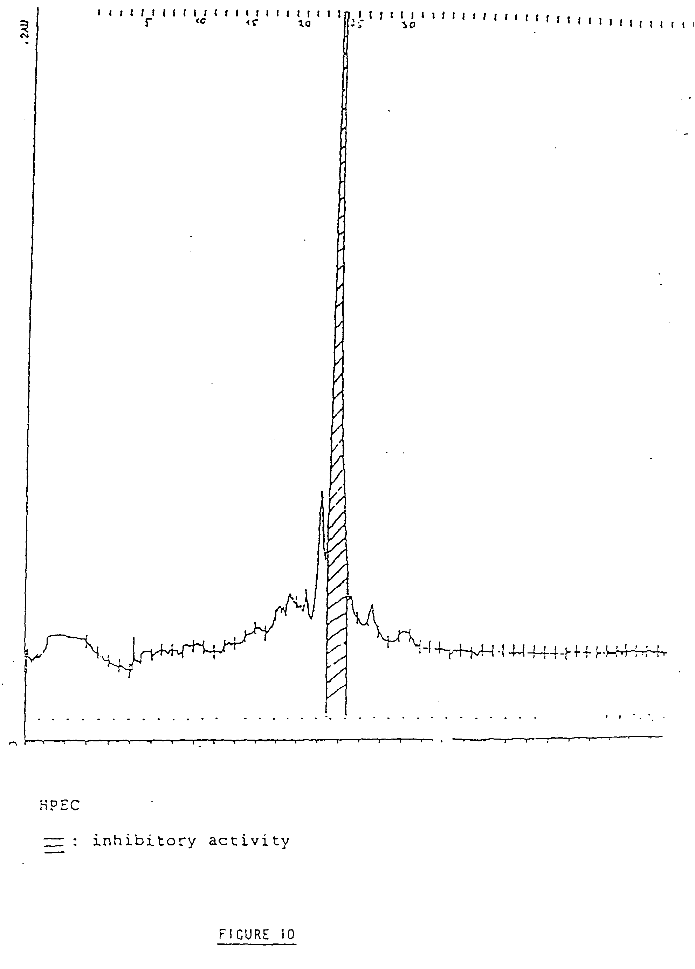

**The protein of the invention isolated according Example 2 is purified to homogeneity by using a High Performance Electrophoresis Chromatography System.**
The partially purified inhibitor is applied to a High Performance Electrophoresis Chromatography System (HPEC) from Applied Biosystems, Inc. (Foster City, CA). The electrophoresis is performed on a 7.5% polyacrylamide-gel in a Tris/glycine buffer system according to the manufacturer's instructions. The sample buffer contained SDS but no reducing agent (e.g., DTT) and the aliquot is not heated prior to being loaded onto the gel. The protein is successively eluted from the gel, detected by measuring the absorption at 230 nm and fractionated. Fractions which have inhibitory activity are analyzed by a SDS-polyacrylamide-gel-electrophoresis (12.5% SDS-polyacrylamide gel, stained with Coomassie Brilliant Blue). See Figure 10.

### Amino acid analysis (Example 16)

Protein samples are evaporated to dryness and hydrolyzed in 6N HCl containing 2% phenol, for 24, 48 and 72 hours. Cysteine content is determined as cysteic acid after performic acid oxidation (MOORE, J. Biol. Chem. **238**, 235-27 (1963)). Tryptophan is measured after hydrolysis in 4N N-methanesulfonic-acid for 24 hours (Simpson et al., J. Biol. Chem. **251**, 1936-1940 (1976)). The samples are then analysed on an amino acid analyzer. The analysis shows the following results (indicated in % of all amino acids): Gly = 8.3%; Ala = 1.6%; Ser = 8.9%; Thr = 10.7%; Val = 8,7%; Leu = 6.6%; Ile = 2.5%; Pro = 4.5%; Cys = 3,0%; Met = 1.0%; His = 2.3%; Tyr = 4.3%; Asp = 6.2%; Glu = 7.2%; Lys = 11.0%; Arg = 1.8%; Asn = 5.7%; Gln = 2.3%; Phe = 2.5%; and Trp = 1.1%.

### Amino acid sequencing (Example 17)

The protein is sequenced on an Applied Biosystems, Inc. (ABI) (Foster City, CA) Automatic Amino Acid Sequenator according to the manufacturer's instructions. The sequence of amino acids 1-20 (from the N-terminus) is: 
Glu-Glu-Cys-Glu-Leu-Met-Pro-Pro-Gly-Asp-Asp-Phe-Asp-Leu-Glu-Lys-Tyr-
1 5 10 15 17

Phe-Ser-Ile
18 20
 **PCR amplification, subcloning and DNA sequencing of major fragments of three forms of inhibitor cDNA from *Triatoma pallidipennis* salivary gland cDNA (Example 18)**

### Part 1. Preparation of Triatoma pallidipennis salivary gland RNA and synthesis of first strand cDNA

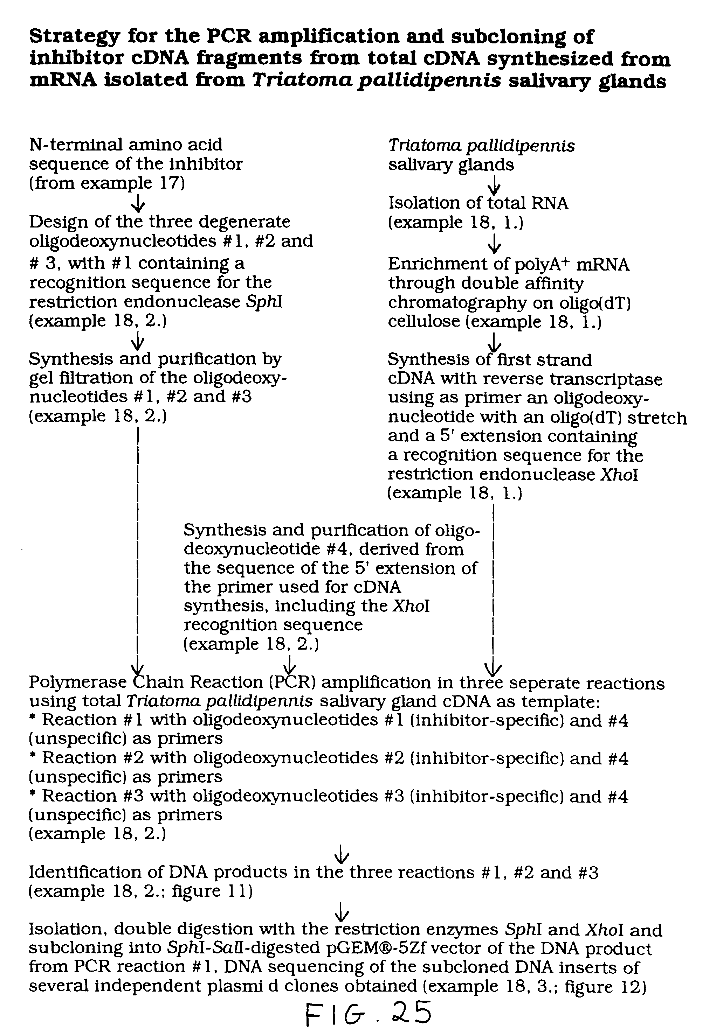

**Starting with the total purified RNA from the salivary glands, the sequences of the RNA are transcribed by the reverse transcriptase to ontain first strand cDNAs. A special oligodeoxynucleotide is used for priming of first strand cDNA synthesis.** Total RNA is isolated from the salivary glands of *Triatoma pallidipennis* in a procedure involving the dissolution of tissue in guanidinium thiocyanate and subsequent ultra-centrifugation of the lysate on a cesium chloride cushion (SAMBROOK, J., FRITSCH, E.F., MANIATIS, T.: Molecular Cloning, Chapter 7, 18-22, Cold Spring Harbor Laboratory Press, 1989). 10 µg of total salivary gland RNA thus obtained are used to synthesize the first strand of complementary DNA (cDNA). For this purpose, Moloney Murine Leukemia Virus reverse transcriptase, the corresponding reaction buffer, deoxynucleotides and RNase block II from a commercially available "1st Strand Synthesis Kit" (Stratagene Cloning Systems, La Jolla, CA, U.S.A.) are used as described in the manufacturer's protocol. The oligodeoxynucleotide incorporated in the annealing step of the reaction for priming first strand cDNA synthesis is not one of those included in the "1st Strand Synthesis Kit" but is a linker-primer (input: 1.4 µg) taken from the commercially available "ZAP-cDNA™ Synthesis Kit" (Stratagene Cloning Systems). Its sequence is as follows (an *Xho*I restriction endonuclease recognition sequence is underlined; see also the summary in Fig. 25)

5'-GAGAGAGAGAGAGAGAGAGAACTAGTCTCGAGTTTTTTTTTTTTTTTTTT -3'
XhoI

### Part 2. PCR amplification of inhibitor cDNA fragments from the salivary gland first strand cDNA

**Taking the amino acid sequence, which is determined by Edman degradation of the purified protein of the invention, three oligodeoxynucleotides and one linker oligodeoxynucleotide are devised and synthesized.** Based on selected stretches of the amino acid sequence determined for the N-terminus of purified inhibitor (Example 17), three degenerated oligodeoxynucleotides are devised and synthesized for the amplification of a major part of inhibitor cDNA. Their sequences are as follows ("I" stands for deoxyinosine, two letters in parentheses divided by a slash indicate positions where two different deoxynucleotides are incorporated, the corresponding amino acid sequence is indicated in three-letter code under the deoxynucleotide sequence, an *Sph*I restriction endonuclease recognition sequence is underlined):

Oligodeoxynucleotide #1:
5'- GCGGC ATG CCI CCI GGI GA(C/T) AA(C/T) TT(C/T) GA -3'
SphI
Met Pro Pro Gly Asp Asn Phe Asp

Oligodeoxynucleotide #2:
5'- AAC TTT GA(C/T) (C/T)TI GA(G/A) AA(G/A) TA(C/T) TT -3'
Asn Phe Asp Leu Glu Lys Tyr Phe

Oligodeoxynucleotide #3:
5'- ATG CCI CCI GGI GA(C/T) AA(C/T) TTT GA(C/T) (C/T)TI GAG AAG
Met Pro Pro Gly Asp Asn Phe Asp Leu Glu Lys
cont.
TA(C/T) TT -3'
Tyr Phe

The oligodeoxynucleotides correspond to different partially overlapping parts of the found amino acid sequence by Edman degradation. Oligodeoxynucleotide #3 comprises the oligodeoxynucleotide #1 in the beginning and the oligodeoxynucleotide #2 in the end.
An additional oligodeoxynucleotide is made with a sequence derived from the linker-primer used for the priming of first strand cDNA synthesis (see Part 1.):

Oligodeoxynucleotide #4:
5'- GAGAGAGAGAACTAGTCTCGAG -3'
XhoI

After synthesis on an Applied Biosystems PCR-Mate™ 391 DNA synthesizer, the four deoxyoligonucleotides are purified through gel filtration on commercially available NAP-5 columns (Pharmacia Biosystems) and used as primers in three separate polymerase chain reactions (PCR; United States Patent 4,800,159) as described below. Reagents from a commercially available "GeneAmp™ DNA Amplification Kit" with *AmpliTaq*™ recombinant *Taq* DNA polymerase from Perkin-Elmer Cetus (Norwalk, CT, USA) are used according to the supplier's protocol. 5% (2.5 µl) of the total amount of the first strand cDNA synthesized from *Triatoma* salivary gland total RNA (see Part 1.) serves as template in each of the three PCR reactions. The oligodeoxynucleotide primers are combined in the following way: oligodeoxynucleotides #1 and #4 in PCR reaction #1, oligodeoxynucleotides #2 and #4 in PCR reaction #2, oligodeoxynucleotides #3 and #4 in PCR reaction #3. The three PCR reactions are incubated in a Perkin Elmer Cetus Thermal DNA Cycler using the following cycling program with 38 cycles comprising cycling steps #1 through #3:
*initial step: 3 min at 94 °C
*cycling program:
   - *cycling step #1:: 1 min 30 sec at 94 °C
   - *cycling step #2:: 2 min at 40 °C
   - *cycling step #3:: 3 min at 72 °C
   (The sequence of cycling steps #1 through #3 is repeated 38 times.)
*final step: 10 min at 72 °C.

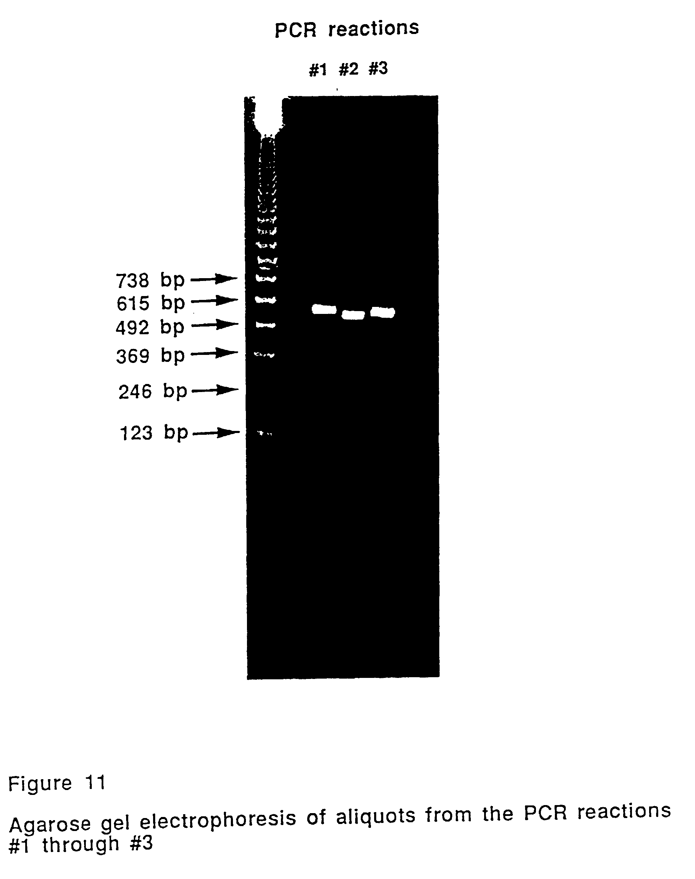

5% (5 µl) of the total reaction volumes are separated through electrophoresis on a 1.5% agarose gel using a 123 base-pair ladder DNA size standard (Gibco-BRL Life Technologies, Gaithersburg, MD, U.S.A.). After staining of the gel with ethidium bromide, single DNA bands are found for each of the three PCR reactions, their apparent sizes according to the DNA size standard being approximately 530 to 560 base pairs (Figure 11, from left to right: 123 base-pair ladder, PCR reactions #1, #2, #3).

### Part 3. Subcloning and sequencing of the inhibitor cDNA fragments

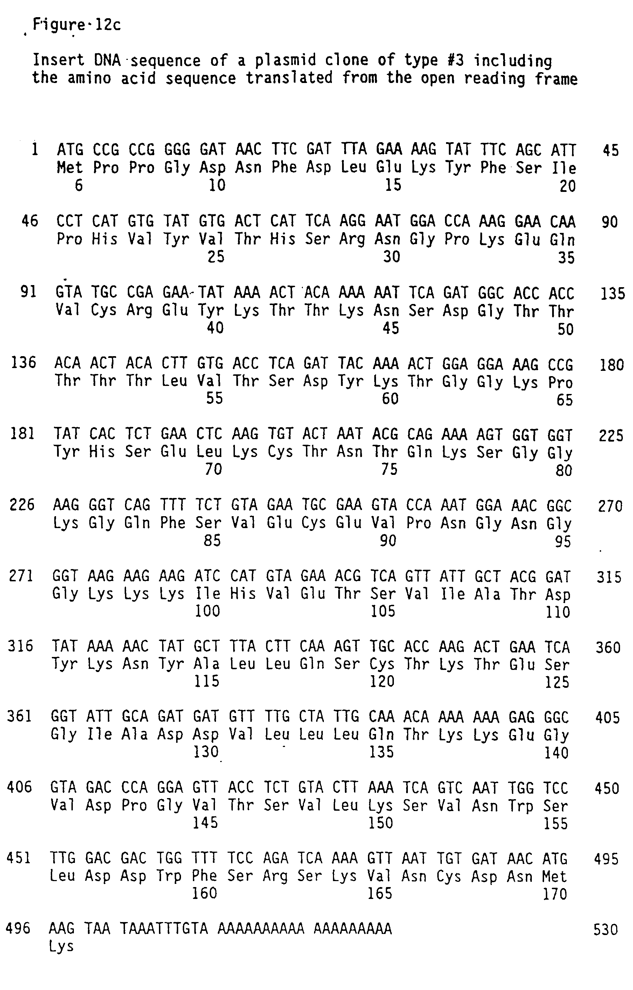

The DNA fragment contained in the remaining volume of PCR reaction #1 (see 2.) is isolated after electrophoresis on a 1.5% agarose gel in a procedure involving binding to and elution of the DNA from NA-45 DEAE membrane (Schleicher & Schuell, D-3354 Dassel, Germany), followed by extraction with n-butanol and ethanol precipitation (SAMBROOK, J., FRITSCH, E.F., MANIATIS, T.: Molecular Cloning, Chapter 6, 24-27, Cold Spring Harbor Laboratory Press, 1989). The ends of the recovered DNA fragment are made suitable for ligation to a vector by double digestion with the restriction enzymes *Sph*I and *Xho*I (Boehringer Mannheim GmbH, D-6800 Mannheim, Germany) and then extracted twice with phenol/chloroform (1:1) and subsequently twice with chloroform. 3 µg of DNA of the plasmid vector pGEM^{R}-5Zf(-) (Promega, Madison, WI, U.S.A.) are linearized through a double digestion using the restriction enzymes *Sph*I and *Sal*I (Boehringer Mannheim GmbH) and then separated on and isolated from a 1.5% agarose gel as described above. 50% of the digested and extracted amplified DNA fragment and 20% of the digested and gel-purified vector DNA are combined, ethanol precipitated and ligated using the reagents and the protocol of a commercially available "DNA Ligation Kit" (Stratagene Cloning Systems). The entire ligation reaction is used for transformation of commercially available "Epicurian Coli^{R}XL1-Blue Supercompetent Cells" (Stratagene Cloning Systems) according to the supplier's protocol. The entire transformation reaction is plated on LB agar plates containing ampicillin (100 µg/ml). 20 of the ampicillin-resistant *E. coli* cell clones found after incuabtion are propagated in LB broth containing ampicillin (100 µg/ml) and their plasmid DNA is isolated in an alkaline lysis "miniprep" procedure (SAMBROOK, J., FRITSCH, E.F. MANIATIS, T.: Molecular Cloning, Chapter 1, 25 - 28, Cold Spring Harbour Laboratory Press, 1989). After double digestion of the plasmid DNA from the 20 clones with the restriction endonucleases *Sph*I and *Sac*I (Boehringer Mannheim GmbH) and electrophoresis on a 1.5 % agarose gel, 13 of them are found to carry a DNA insert of an apparent size of approximately 580 bp ("positive clones"). DNA sequencing is perfomed on the phenol/chloroform-extracted plasmid DNA of 5 of theses positive clones using a commercially available "Sequenase^{R} Version 2.0 DNA Sequencing Kit" (United States Biochemical Corporation, Cleveland, OH. USA) after priming with both T7 or SP6 primers (Promega). The complete insert sequences of the different plasmids is determined in this way. A single open reading frame can be identified in each of the five insert sequences. Three types of insert sequences are found with respect to the amino acid sequences derived from the open reading frame, three of the sequenced plasmid clones belonging to the type #1 and one each to the type #2 and type #3 of the derived amino acid sequence. The complete DNA insert sequences of one representative of each of the three plasmid types #1 through #3 are depicted in Figure 12 together with the amino acid sequence translated from the open reading frame. The sequence of the first 15 amino acids derived from each type of plasmid insert is identical to that determined for amino acid position 6 to 20 of the N-terminus of the inhibitor isolated from *Triatoma* saliva (Example 17).

### Locating, isolating and cloning a gene coding for a platelet aggregation inhibitor (Example 19)

A genomic library containing cloned restriction fragments from arestriction endonuclease digests of *T. pallidipennis* DNA is screened with the probes, on replicate filter lifts according to standard methods. Clones which hybridize with the probe are selected. The DNA insert from these clones is then further subcloned according to standard methods until a minimum-sized DNA is isolated which binds to the probe.

This fragments are sequenced and then transferred into a suitable eucaryotic expression vector by inserting the coding region into an expression vector containing all of the elements reqired for expression, e.g., a promoter sequence, a terminator sequence and an origin of replication, all of which are operably linked to the PAI gene (PAI = platelet aggregation inhibitor), as well as a selection marker for isolating the thus-formed expression vector. The expression vector is then transformed into the eukaryotic host for which it is designed, and the PAI expression product is isolated.

### Sequencing the gene coding for platelet aggregation inhibitor (PAI) (Example 20)

Single- and double-stranded DNA sequencing is carried out using the dideoxynucleotide chain termination method as described in SANGER et al., Proc. Natl. Acad. Sci. USA (1977) **74**, 5463-5467.

### Screening genomic libraries and mutant clones for new sequences related to the T. pallidipennis sequence (Example 21)

As above, the probes derived from the amino acid sequence of the inhibitor isolated from *T. pallidipennis* are used to screen other genomic libraries for sequences related to the present inhibitor. Similarly, libraries of mutant inhibitors, produced by routine mutagenesis of vectors containing the gene for the *T. pallidipennis* inhibitor as produced above with NNMG and by site directed mutagenesis, are screened for activity.

### Isolation, characterization and sequencing of complete cDNA clones for two proteins of invention as isoforms (Example 22)

### a) General Approach

A cDNA library derived from polyA(+) RNA extracted from *T. pallidipennis* is screened with the probe of Example 18 on replicate filter lifts according to standard methods. Positive clones are purified by separate plating out and repetition of the plaque filter hybridization. The cDNA of the longest cDNA clones are sequenced by the dideoxynucleotide method of Sanger.

### b) Concrete description of the assays

### Part 1. Construction of a cDNA library from Triatoma pallidipennis salivary gland RNA

Approximately 500 µg of total RNA isolated from *Triatoma pallidipennis* salivary glands as described above (Example 18, Part 1.) are used for the isolation of polyA⁺ mRNA through double affinity chromatography on oligo(dT)-cellulose. For this purpose, a commercial available "mRNA Purification Kit" (Pharmacia Biosystems GmbH, W-7800 Freiburg, Germany) is used for two subsequent rounds of enrichment as described in the manufacturer's instructions. The final yield after the second purification step is 13 µg of polyA⁺ mRNA. 5 µg of this preparation is used for the construction of a cDNA library in the "Lambda ZAP® II" bacteriophage vector with the reagents and procedures of the commercially available "ZAP-cDNA™ Gigapack® II Gold Cloning Kit" (Stratagene Cloning Systems). 33% of the final yield of the first cDNA fraction after size-fractioning are ligated to 2 µg of the bacteriophage vector DNA. After packaging of the entire ligation reaction in 7 separate packaging reactions, an unamplified cDNA library with a total of 20 · 10⁶ independent recombinant phages is obtained.

### Part 2. Isolation of the inhibitor cDNA clones from the Triatoma pallidipennis salivary cDNA library

A total of 5 · 10⁵ recombinant phages from the cDNA library described above (1.) are screened through DNA-DNA hybridization of double plaque lifts on Biodyne® A nylon membranes (Pall BioSupport, East Hills, NY, U.S.A.). Hybridization is carried out in a solution containing 5 x SSC, 5 x Denhardt's solution, 0.2% SDS and 100 µg/ml of denatured phenol-extracted sonicated salmon sperm DNA (Sigma Chemical Company, St. Louis, MO, U.S.A.) with a radiolabeled DNA probe prepared as described below. The insert DNA of a plasmid clone of type #1 from Example 18 is isolated after double digestion using the restriction endonucleases *Sph*I and *Sac*I as described above. Approximately 25 ng of the recovered insert DNA are radiolabeled using a "Prime-IT™ Random Primer Labeling Kit" (Stratagene Cloning Systems) in the presence of [α-³²P] dCTP (3000 Ci/mmol; Amersham Buchler, W-3300 Braunschweig, Germany). The labeled DNA fragment is separated from unincorporated radioactivity by chromatography on a "NAP™-5" column (Pharmacia Biosystems). Filter hybridization and washing temperature is 50 °C, the final wash step is in 2 x SSC with 0.2% SDS. After autoradiography at -70 °C for 48 hours, more than 300 plaques are found to yield signals on both replica filters. The bacteriophages from 80 areas around such positive signals are eluted from the original overlay plate and replated separately, at a density allowing for the purification of single phage clones. The plaque hybridization procedure described above is repeated using the same DNA probe and a total of 76 independent phage clones giving positive signals are isolated from the plates.

### Part 3. Characterization and sequencing of the inhibitor cDNA clones

The phage clones are separately subjected to the "*in vivo* excision" procedure described in the protocol of Stratagene's cDNA library construction kit referred to above. "Miniprep" plasmid DNA from 76 different pBluescript SK plasmid clones isolated after the "*in vivo* excision" is cleaved in a double digestion with the restriction endonucleases *Eco*RI and *Xho*I (Boehringer Mannheim), separated on a 1.5% agarose gel and stained with ethidium bromide. A variety of different insert sizes ranging up to approximately 620 base pairs is observed.

DNA sequencing with T3 and T7 primers (Stratagene Cloning Systems) is performed as described above on the plasmid DNA of 8 clones that are found to carry the largest DNA inserts of all 76 independent clones investigated. cDNA clones are thus identified that belong to 2 classes according to the amino acid sequence translated from the open reading frame, one class corresponding exactly to the type #1 plasmid insert (6 clones, called "inhibitor-1") described in example 18 (3), and the other class to the type #2 plasmid insert (2 clones, called "inhibitor-2"). Further DNA sequencing experiments are carried out to confirm the sequences established so far, using additional synthetic oligodeoxynucleotides based on the known sequences:

Oligodeoxynucleotide #5:
5'- TATCACTCTGAACTCAAGTG -3'

Oligodeoxynucleotide #6:
5'- TTACCGCCGTTTCCATTTGG -3'

Oligodeoxynucleotide #7:
5'- TTACTTCAAAGTTGCACC -3'

Oligodeoxynucleotide #8:
5'- GCAACATGAAGGTGATCATTGCAGCAAC -3'

The 5' ends of most of the longest independent clones are found to be identical, with a 5'-untranslated region of 5 base pairs, suggesting that the cDNAs of the complete mRNA transcripts including the transcription initiation point have been cloned. The complete DNA and derived amino acid sequences of the cDNA clones for inhibitor-1 and inhibitor-2 are depicted in Figure 13. The cleavage site between signal peptide and mature protein is deduced from the N-terminal amino acid sequence decribed in Example 17.

### Expression and secretion of recombinant inhibitor in stably transfected baby hamster kidney (BHK) cells (Example 23)

### a) General approach

The coding sequence from the cDNA clones is then transferred into a suitable eukaryotic expression vector by inserting the coding region into an expression vector containing all of the elements required for expression, e.g., a promoter sequence, a terminator sequence and an origin of replication, all of which are operably linked to the PAI gene, as well as a selection marker for isolating the thus-formed expression vector. The expression vector is then transformed into the eukaryotic host for which it is designed, and the PAI expression product is isolated.

### b) Concrete description of the assays

### Part 1. Construction of expression plasmids for the inhibitor using the pMPSV/CMV vector

Two oligodeoxynucleotides are synthesized for the PCR amplification of inhibitor-coding sequences from both inhibitor-1 and inhibitor-2 plasmid cDNA clones. One of them (#9) is deduced from the coding strand of the region around the ATG initiation codon, prolonged with a 5' tail including a *Hin*dIII recognition sequence and an optimized "Kozak site" (KOZAK, M.: Point mutations define a sequence flanking the AUG initiation codon that modulates translation by eukaryotic ribosomes. Cell **44**, 283-292, 1986), while the other (#10) is deduced from the non-coding strand of the region around the TAA termination codon of the open reading frames, prolonged with a 5' tail including a *Hin*dIII recognition sequence (recognition sequences of the restriction endonuclease *Hin*dIII are underlined, the optimized "Kozak site" for efficient translation initiation is indicated with asterisks, the portions of the sequences matching one or the other strand of the original cDNA clone sequences are in italics):

Oligodeoxynucleotide #9:
5'- GCGATAAAGCTTCCACCATGAAGGTGATCATTGCAGC -3'
HindIII *********

Oligodeoxynucleotide #10:
5'- GCGATAAAGCTTATTACTTCATGTTATCAC -3'
HindIII

Using approximately 3 µg of either cDNA clone plasmid as template, two separate PCR amplifications are carried out in the presence of the two oligodeoxynucleotide primers #9 and #10 as described above (example 18, Part 2.), with however 18 instead of 38 cycles comprising cycling steps #1 through #3. The amplified coding sequences of inhibitor-1 and inhibitor 2, which carry the optimized Kozak site but lack a complete polyadenylation signal (5'-....AATAAA..... -3') found immediately 3' of the termination codon of the original cDNA clones, are then isolated and made suitable for ligation through digestion with the restriction endonuclease *Hin*dIII (Boehringer Mannheim) and subsequent extraction steps as described above (example 18, Part 3.). 3 µg of plasmid DNA of the pMPSV/CMV-HE vector (WIRTH, M., SCHUMACHER, L., HAUSER, H.: Construction of new expression vectors for mammalian cells using the immediate early enhancer of the human cytomegalovirus to increase expression from heterologous enhancer/promoters. In: CONRADT, H.S. [Ed.], Protein Glycosylation: Cellular, Biotechnical and Analytical Aspects. Vol. 15, 49-52, VCH publishers, Weinheim, 1991; KRÄTZSCHMAR, J., HAENDLER, B., BRINGMANN, P. DINTER, H. HESS, H., DONNER, P., SCHLEUNING, W.-D.: High-level secretion of the four salivary plasminogen activators from the vampire bat *Desmodus rotundus* by stably-transfected baby hamster kidney cells. Gene, (1992) **116**; 281- 284 are linearized through digestion with the restriction endonuclease *Hin*dIII and isolated as described. The recovered plasmid DNA is dephosphorylated using 1 unit of calf intestinal alkaline phosphatase (Boehringer Mannheim), subjected to the extraction procedure and then used for subcloning of the inhibitor-coding PCR fragments as described in example 18, Part 3..

The DNA of the obtained pMPSV/CMV-inhibitor-1 or -2 constructs carrying *Hin*dIII inserts is digested with the restriction endonuclease *Eco*RI (Boehringer Mannheim) and in about half of the cases, an *Eco*RI restriction fragment of about 580 base pairs is seen, indicating those constructs where the inhibitor-coding insert is in the correct orientation with respect to the Myeloproliferative Sarcoma Virus promoter of the pMPSV/CMV vector. The complete inhibitor-coding inserts of such pMPSV/CMV-inhibitor-1 and -2 constructs are then sequenced using oligodeoxynucleotides #5 through #8 and two additional primers (oligodeoxynecleotides #11 and #12) derived from the insert-flanking region of the expression vector to check for mutations that might have been introduced during PCR amplification:

Oligodeoxynucleotide #11:
5'- ACCAGAAAGTTAACTGG -3'

Oligodeoxynucleotide #12:
5'- CCTAGTTTGTGGTTGTCC -3'

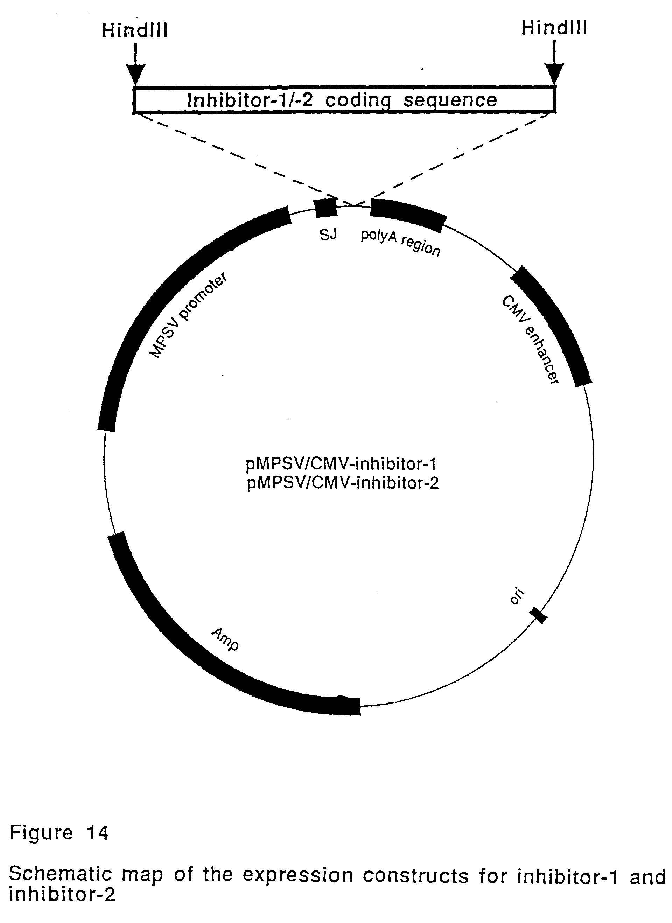

Two constructs for expression of inhibitor-1 and inhibitor-2 in mammalian cells coding for proteins identical in their amino acid sequence to those depicted in Figure 13 are thus obtained. A schematic map of the constructs is given in Figure 14 ("Amp": ampicillin-resistance marker, "MPSV promoter": Myeloproliferative Sarcoma Virus promoter, "SJ": SV40 intron including its splice junctions, "polyA region": SV40 polyadenylation region, "CMV enhancer": cytomegalovirus enhancer, "ori": pBR322 origin of replication).

### Part 2. Transfection and selection of BHK cells

Plasmid DNA of two resequenced pMPSV/CMV-inhibitor-1 and -2 constructs is isolated using "Qiagen-tip 100" columns (Qiagen Inc. Chatsworth, CA, U.S.A.). Likewise, two plasmids that carry resistance marker genes, one for hygromycin B kinase (pSK/HMR272, constructed through subcloning of a *BamH*I-*Hin*dIII fragment containing the HSVtk promoter linked to the hygromycin B kinase gene into BluescriptSK, which fragment is taken from the pHMR272 vector described in: BERNHARD, H.U., KRÄMMER, G., RÖWEKAMP, W.G.:Construction of a fusion gene that confers resistance against hygromycin B to mammalian cells in culture, Experimentl Cell Research **158**, 237-243, 1985) and the other for puromycin-N-acetyltransferase (pSV2pacΔp; DE LA LUNA, S., SORIA, I., PULIDO, D., ORTIN, J.,JIMENIEZ, A.: Efficient transformation of mammalian cells with constructs containing a puromycin-resistance marker, Gene **62****,** 121-126, 1988), are prepared. Approximately 20 µg of the inhibitor-1 or -2 expression construct, 6 µg of the puromycin-resistance plasmid and 2 µg of the hygromycin-resistance plasmid are used for transfection of baby hamster kidney (BHK) cells as described (KRÄTZSCHMAR, J., HAENDLER, B., BRINGMANN, P., DINTER, H., HESS, H., DONNER, P., SCHLEUNING, W.-D.: (1992) High-level secretion of the four salivary plasminogen activators from the vampire *Desmodus rotundus* by stably-transfected baby hamster kidney cells. Gene, **116;** 281 - 284) using "Lipofectin™ Reagent" (Gibco-BRL Life Technologies). A double selection procedure is applied using DMEM/10% FCS (Gibco-BRL Life Technologies) containing 0.7 mg/ml of hygromycin B (Calbiochem Corporation, La Jolla, CA, U.S.A.) and 5 µg/ml of puromycin (Sigma Chemical Company). The mixtures of double resistant BHK cells transfected with pMPSV/CMV-inhibitor-1 or -2 obtained after two weeks of selection are grown in serum-free OPTI-MEM (Gibco-BRL Life Technologies) as described (KRÄTZSCHMAR, J., HAENDLER, B., BRINGMANN, P., DINTER, H., HESS, H., DONNER, P., SCHLEUNING, W.-D.: High-level secretion of the four salivary plasminogen activators from the vampire *Desmodus rotundus* by stably-transfected baby hamster kidney cells. Gene, (1992); **116;** 281 - 284) The conditioned media are collected after 24 hours, freed from cell debris through centrifugation at 2000 x g and stored frozen.

### Part 3. Detection of recombinant inhibitor in BHK cell culture supernatants

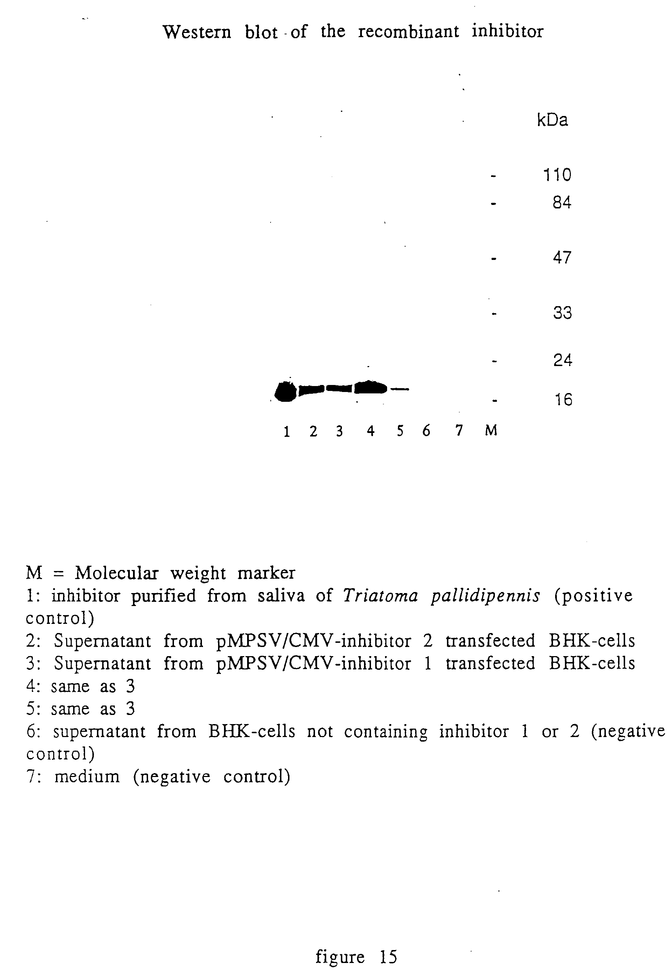

Aliquots of the conditioned media are tested for inhibitor production in a Western blot (see Example 24). The anti-inhibitor antiserum reacts specifically with a 19 kDa protein present in the conditioned medium from pMPSV/CMV-inhibitor-1-transfected BHK cells from pMPSV/CMV-inhibitor-2-transfected BHK cells. No reaction is observed with control media from cells transfected with a pMPSV/CMV construct not containing the inhibitor insert or with fresh control medium (see Figure 15). Extracts of the transfected cells give only a faint signal indicating that the recombinant proteins are secreted into the medium. Beside the immunological detection of the two recombinant inhibitor forms, the supernatants of the transfected BHK cells can also be tested in a functional assay. The inhibition of collagen-induced platelet aggregation can be measured in an aggregation assay as described in example 1.

### Antibody production (Example 24)

About 100 µg of the inhibitor purified according to example 2 and 15 are added to 0.5 ml of complete Freund's adjuvant and the emulsion is injected s.c. into a rabbit. After 2 weeks a second injection is given consisting of about 80 µg purified inhibitor and 0.5 ml incomplete Freund's adjuvant. After the injection, several samples of serum are taken to check the production of specific antibodies. They are assayed in a Western blot. 20 ng of the purified inhibitor is applied on a 12.5% SDS-polyacrylamide gel and the electrophoresis, blotting and detection are done according to standard methods described by E. HARLOWE, D. LANE, (1988) Antibodies: a laboratory manual, Cold Spring Harbour Laboratory (dilution of the test serum 1:500, goat anti rabbit peroxidase conjugated IgG as second antibody, detection with the ECL-kit from Amersham International, Amersham, UK). The blot shows that the antiserum specifically reacts with the purified inhibitor.

## Claims

1. A protein which inhibits collagen-induced aggregation of mammalian platelets having the following amino acid sequence:
a) the sequence in
aa) Seq. Id. No. 1,
bb) Seq. Id. No. 2 or
cc) Seq. Id. No. 3
or
b) allelic modifications or muteins of the sequences in anyone of the Seq. Id Nos. 1 to 3, which allelic modifications or muteins do not substantially affect the activity of the protein,
or
c) a protein according to anyone of the Seq. Id Nos. 1 to 3 or their modifications or muteins mentioned under b), and further comprising posttranslational modifications which do not substantially affect the activity of the mature protein.

2. A protein according to claim 1, which is recombinantly produced.

3. A protein according to claims 2, which is unglycosylated.

4. A cDNA or DNA
a) coding for a protein having the following amino acid sequence:
a) the sequence in
aa) Seq. Id. No. 1,
bb) Seq. Id. No. 2 or
cc) Seq. Id. No. 3
or
b) coding for a protein having a sequence of amino acids according to anyone of the Seq. Id Nos. 1 to 3, having at least one allelic modification or mutein which does not substantially affect the activity of the mature protein encoded by the corresponding cDNA or DNA sequence.

5. A cDNA or DNA having the following nucleotide sequence:
a) the nucleotide sequence in
aa) Seq. Id. No. 4,
bb) Seq. Id. No. 5 or
cc) Seq. Id. No. 6
or
b) a sequence of nucleotides according to any one of the Seq. Id Nos. 4 to 6, having at least one allelic modification or mutein which does not substantially affect the activity of the mature protein, which is encoded by the corresponding nucleotide sequence.

6. A vector comprising a cDNA or DNA according to claim 4 or 5, a suitable signal peptide, a suitable promoter and, if necessary, a suitable enhancer.

7. An eukaryotic or prokaryotic host cell transformed with a vector according to claim 6.

8. A host cell according to claim 7, which is a baby hamster kidney cell.

9. A method of producing a protein according to anyone of the claims 1 to 3, comprising
culturing a host cell transformed by a vector comprising the gene coding for the protein and
isolating and purifying the protein.

10. A method of purifying a protein according to anyone of the claims 1 to 3 comprising
(a) separating the protein on a gel filtration column using "Superose 12" and
(b) eluting the protein on a high performance electrophoresis chromatography system.

11. A pharmaceutical composition comprising a protein according to anyone of the claims 1 to 3 and a pharmaceutically acceptable diluent or carrier.

12. Use of a protein according to anyone of the claims 1 to 3 for the production of a medicament for a therapeutic application for inhibiting collagen-induced human platelet aggregation prophylactically, or in an acute situation.

13. Use of a protein according to anyone of the claims 1 to 3 for the production of a medicament for the treatment of cancer with metastatic tumor cells.

## Patentansprüche

1. Protein, das die collageninduzierte Anhäufung von Säugerblutplättchen hemmt und die folgende Aminosäuresequenz aufweist:
a) die Sequenz in
aa) Seq. Id. Nr. 1,
bb) Seq. Id. Nr. 2 oder
cc) Seq. Id. Nr. 3
oder
b) allele Modifikationen oder mutierte Proteine der Sequenzen in einer der Seq. Id. Nr. 1 bis 3, wobei die allelen Modifikationen oder mutierten Proteine die Aktivität des Proteins nicht wesentlich beeinflussen,
oder
c) ein Protein nach einer der Seq. Id. Nr. 1 bis 3 oder ihre unter b) erwähnten Modifikationen oder mutierten Proteine, weiterhin umfassend posttranslationelle Modifikationen, die die Aktivität des reifen Proteins nicht wesentlich beeinflussen.

2. Protein nach Anspruch 1, das rekombinant hergestellt wird.

3. Protein nach Anspruch 2, das nicht glycolysiert ist.

4. cDNA oder DNA,
a) die für ein Protein mit der folgenden Aminosäuresequenz kodiert:
a) die Sequenz in
aa) Seq. Id. Nr. 1,
bb) Seq. Id. Nr. 2 oder
cc) Seq. Id. Nr. 3
oder
b) die für ein Protein kodiert, das eine Sequenz von Aminosäuren gemäß einer der Seq. Id. Nr. 1 bis 3 aufweist, die mindestens eine allele Modifikation oder ein mutiertes Protein aufweist, welche bzw. welches die Aktivität des reifen Proteins, das durch die entsprechende cDNA- oder DNA-Sequenz kodiert wird, nicht wesentlich beeinflußt.

5. cDNA oder DNA mit der folgenden Nucleotidsequenz:
a) die Nucleotidsequenz in
aa) Seq. Id. Nr. 4,
bb) Seq. Id. Nr. 5 oder
cc) Seq. Id. Nr. 6
oder
b) eine Sequenz von Nucleotiden gemäß einer der Seq. Id. Nr. 4 bis 6, die mindestens eine allele Modifikation oder ein mutiertes Protein aufweist, die bzw. das die Aktivität des reifen Proteins nicht wesentlich beeinflußt, das durch die entsprechende Nucleotidsequenz kodiert wird.

6. Vektor, der eine cDNA oder DNA nach Anspruch 4 oder 5, ein geeignetes Signalpeptid, einen geeigneten Promotor und, falls notwendig, einen geeigneten Enhancer umfaßt.

7. Mit einem Vektor nach Anspruch 6 transformierte, eukaryontische oder prokaryontische Wirtszelle.

8. Wirtszelle nach Anspruch 7, die eine Babyhamsternierenzelle ist.

9. Verfahren zur Herstellung eines Proteins nach einem der Ansprüche 1 bis 3, umfassend
Züchten einer Wirtszelle, die durch einen das für das Protein kodierende Gen umfassenden Vektor transformiert wird und
Isolieren und Reinigen des Proteins.

10. Verfahren zur Reinigung eines Proteins nach einem der Ansprüche 1 bis 3, umfassend
a) Abtrennen des Proteins an einer Gelfiltrationsssäule unter Verwendung von "Superose 12" und
b) Eluieren des Proteins an einem Hochleistungs-Elektrophoresechromatographiesystem.

11. Pharmazeutische Zusammensetzung, umfassend ein Protein nach einem der Ansprüche 1 bis 3 und ein pharmazeutisch annehmbares Verdünnungsmittel oder einen pharmazeutisch annehmbaren Träger.

12. Verwendung eines Proteins nach einem der Ansprüche 1 bis 3 für die Herstellung eines Medikaments für eine therapeutische Anwendung zum Hemmen der collageninduzierten Blutplättchenansammlung beim Menschen als Prophylaxe oder in einer Akutsituation.

13. Verwendung eines Proteins nach einem der Ansprüche 1 bis 3 für die Herstellung eines Medikaments für die Behandlung von Krebs mit metastasierenden Tumorzellen.

## Revendications

1. Protéine qui inhibe l'agrégation induite par le collagène de plaquettes mammifères ayant la séquence d'acides aminés suivante :
a) la séquence dans
aa) Seq. Id. No. 1,
bb) Seq. Id. No. 2 ou
cc) Seq. Id. No. 3
ou
b) des modifications alléliques ou mutéines des séquences suivant l'une quelconque des Seq. Id Nos. 1 à 3, lesquelles modifications alléliques ou mutéines n'influencent pratiquement pas l'activité de la protéine, ou
c) une protéine suivant l'une quelconque des Seq. Id Nos. 1 à 3 ou leurs modifications ou mutéines mentionnées sous b), et de plus comprenant des modifications posttraductionnelles qui n'influencent pratiquement pas l'activité de la protéine mature.

2. Protéine suivant la revendication 1, qui est produite de façon recombinante.

3. Protéine suivant la revendication 2, qui est non glycosylée.

4. ADNc ou ADN
a) codant pour une protéine ayant la séquence d'acides aminés suivante :
a) la séquence dans
aa) Seq. Id. No. 1,
bb) Seq. Id. No. 2 ou
cc) Seq. Id. No. 3
ou
b) codant pour une protéine ayant une séquence d'acides aminés suivant l'une quelconque des Seq. Id Nos 1 à 3, ayant au moins une modification allélique ou mutéine qui n'influence pratiquement pas l'activité de la protéine mature codée par la séquence d'ADNc ou d'ADN correspondante.

5. ADNc ou ADN ayant la séquence nucléotidique suivante :
a) la séquence nucléotidique dans
aa) Seq. Id. No. 4,
bb) Seq. Id. No. 5 ou
cc) Seq. Id. No. 6
ou
b) une séquence de nucléotides suivant l'une quelconque des Seq. Id Nos. 4 à 6, ayant au moins une modification allélique ou mutéine qui n'influence pratiquement pas l'activité de la protéine mature, qui est codée par la séquence nucléotidique correspondante.

6. Vecteur comprenant un ADNc ou ADN suivant l'une ou l'autre des revendications 4 et 5, un peptide signal approprié, un promoteur approprié et, si nécessaire, un activateur approprié.

7. Cellule hôte eucaryotique ou procaryotique transformée avec un vecteur suivant la revendication 6.

8. Cellule hôte suivant la revendication 7, qui est une cellule de rein de bébé hamster.

9. Procédé de production d'une protéine suivant l'une quelconque des revendications 1 à 3, comprenant :
la culture d'une cellule hôte transformée par un vecteur comprenant le gène codant pour la protéine, et
l'isolement et la purification de la protéine.

10. Procédé de purification d'une protéine suivant l'une quelconque des revendications 1 à 3, comprenant :
(a) la séparation de la protéine sur une colonne de filtration de gel utilisant du "Superose 12", et
(b) l'élution de la protéine sur un système de chromatographie par électrophorèse à haute performance.

11. Composition pharmaceutique comprenant une protéine suivant l'une quelconque des revendications 1 à 3 et un diluant ou support pharmaceutiquement acceptable.

12. Utilisation d'une protéine suivant l'une quelconque des revendications 1 à 3 pour la production d'un médicament pour une application thérapeutique pour inhiber l'agrégation de plaquettes humaines induite par le collagène prophylactiquement ou dans une situation aiguë.

13. Utilisation d'une protéine suivant l'une quelconque des revendications 1 à 3 pour la production d'un médicament pour le traitement de cancer avec des cellules tumorales métastatiques.
